# EUROPÄISCHE PATENTSCHRIFT

(11) **EP 2 467 355 B1**
(45) Veröffentlichungstag und Bekanntmachung des Hinweises auf die Patenterteilung: **03.07.2013**
(21) Anmeldenummer: 10744931.6
(22) Anmeldetag: 19.08.2010
(51) Int. Cl.: C07C 51/235

(54) **VERFAHREN ZUR HERSTELLUNG VON 4-PENTENSÄURE**
METHOD FOR PRODUCING 4-PENTENOIC ACID
PROCÉDÉ DE PRODUCTION D'ACIDE PENTÈNE-4-OÏQUE

(30) Priorität: 21.08.2009 EP 09168353
(43) Veröffentlichungstag der Anmeldung: 27.06.2012
(73) Patentinhaber: BASF SE, 67056 Ludwigshafen (DE)
(72) Erfinder: TELES, Joaquim Henrique, 67166 Otterstadt (DE); SCHELPER, Michael, 67065 Ludwigshafen (DE); GUMLICH, Kai, 68159 Mannheim (DE); CHABANAS, Mathieu, F-67630 Niederlauterbach (FR); MÜLLER, Christian, 68167 Mannheim (DE); MEIER, Anton, 67134 Birkenheide (DE)
(86) Internationale Anmeldenummer: PCT/EP2010/062098
(87) Internationale Veröffentlichungsnummer: WO 2011/020878

(56) Entgegenhaltungen:
- WO-A2-2010/023211
- DE-B1- 2 517 447
- GB-A- 782 430
- N. I. KAPUSTINA ET AL: "Oxidation of Secondary Cyclic Alcohols by Pb(OAc)4 catalyzed by Cu(II) compounds", RUSSION CHEMICAL BULLETIN, Bd. 37, Nr. 10, 1989, Seiten 2095-2099, XP002634795, DOI: 10.1007/BF00953412
- E. V. STAROKON' ET AL: "High-temperature carboxidation of cyclopentene with nitrous oxide", KINETICS AND CATALYSIS, Bd. 48, Nr. 3, 2007, Seiten 376-380, XP002634796, in der Anmeldung erwähnt
- DATABASE CA [Online] CHEMICAL ABSTRACTS SERVICE, COLUMBUS, OHIO, US; XP002634803, gefunden im STN Database accession no. 150:471325 & XIE, JIANCHUN ET AL: "Synthesis of flavor 2- pentenoic acid", SHIPIN KEXUE (BEIJING, CHINA) , 28(10), 252-254 CODEN: SPKHD5; ISSN: 1002-6630, 2007, XP002634797,

## Beschreibung

Die vorliegende Erfindung betrifft ein Verfahren zur Herstellung von 4-Pentensäure, mindestens umfassend die Oxidation eines Gemischs (G) enthaltend 4-Pentenal, 3-Methyl-2-butanon und Cyclopentenoxid sowie die Verwendung eines Gemischs (G) enthaltend 4-Pentenal, 3-Methyl-2-butanon und Cyclopentenoxid zur Herstellung von 4-Pentensäure. Bevorzugt wird im Rahmen der vorliegenden Erfindung das Gemisch (G) als Nebenprodukt der Oxidation von Cyclopenten zu Cyclopentanon mittels Distickstoffmonoxid erhalten.

4-Pentensäure und deren Ester finden als Riech- und Geschmacksstoffe Verwendung, insbesondere in Milch- und Käse-Produkten. Außerdem sind 4-Pentensäure bzw. 4-Pentenoate auch als pharmakologisch aktive Substanzen bekannt, die in der Lage sind, Hypoglykämie zu induzieren (siehe z.B. H. Sherratt, H. Osmundsen, Biochemical Pharmacology (1976) 25(7), 743-750).

Aus dem Stand der Technik ist bekannt, dass 4-Pentensäure durch Umsetzung von Diethylmalonat mit Allylchlorid in Anwesenheit einer Base und anschließender Verseifung, Decarboxylierung und Ansäuerung hergestellt werden kann (siehe J. Xie, B. Sun, S. Sha, F. Zheng, W. Dang, Beijing Gongshang Daxue Xuebao, Ziran Kexueban (2007), 25(1), 7-9). Diese Synthese hat allerdings den Nachteil, dass die Reaktion von relativ teuren Einsatzstoffen ausgeht und diese nur schlecht ausnutzt (Verlust von CO₂). Bei einer beschriebenen Ausbeute von ca. 70% werden etwa 340 kg Edukte, nämlich Diethylmalonat und Allylchlorid, benötigt, um 100 kg 4-Pentensäure herzustellen.

Aus dem Stand der Technik ist weiterhin grundsätzlich die Oxidation von ungesättigten Aldehyden mit Sauerstoff zu den entsprechenden Carbonsäuren bekannt. So ist beispielsweise die Oxidation von alpha,beta-ungesättigten Aldehyden beschrieben. So beschreibt zum Beispiel die WO 2008/017342 die Oxidation von Citronellal zu Citronellsäure mit Sauerstoff in Anwesenheit eines goldhaltigen geträgerten Katalysators. Die US 4,097,523 beschreibt die Oxidation von alpha,beta-ungesättigten Aldehyden mit Sauerstoff in Anwesenheit von Thallium als Katalysator. Andere homogene Katalysatoren wie beispielsweise Mangan, Kupfer oder Cobalt sind auch in der Oxidation von alpha,beta-ungesättigten Aldehyden mit Sauerstoff bekannt ("Crotonaldehyde and Crotonic acid", R. P. Schulz, J. Blumenstein, C. Kohlpaintner in "Ullmann's Encyclopedia of Industrial Chemistry", 7. Ausgabe, online Release 2008). Keine dieser Schriften aus dem Stand der Technik liefert allerdings einen Hinweis darauf, dass diese Methoden zur Oxidation von alpha,beta-ungesättigten Aldehyden auf andere ungesättigte Aldehyde übertragbar wäre.

Ausgehend von diesem Stand der Technik lag eine Aufgabe der vorliegenden Erfindung darin, ein wirtschaftlich interessantes Verfahren zur Herstellung von 4-Pentensäure bereitzustellen.

Es wurde überraschenderweise gefunden, dass 4-Pentensäure durch die Oxidation von 4-Pentenal in guten Ausbeuten und hoher Reinheit hergestellt werden kann.

Ein wesentlicher Faktor der Wirtschaftlichkeit des Oxidationsverfahrens ist die Verfügbarkeit von 4-Pentenal. 4-Pentenal kann beispielsweise durch Claisen-Isomerisierung von Allylvinylether hergestellt werden (siehe z.B. R. F. Webb, A. J. Duke, J. A. Parsons, J. Chem. Soc. (1961), 4092-4095). Allerdings ist der Allylvinylether kein handelsüblicher Vinylether und somit auch technisch nicht leicht verfügbar.

Alternativ kann 4-Pentenal auch durch die Thermolyse von Acetaldehyddiallylacetal hergestellt werden wie in der DE 25 17 447 beschrieben. Allerdings hat diese Methode den Nachteil, dass Allylalkohol rückgeführt werden muss, wobei sich ein nennenswerter Anteil davon zu Propionaldehyd zersetzt, was das Verfahren wiederum unwirschaftlich macht.

Aus dem Stand der Technik ist weiterhin bekannt, dass sich 4-Pentenal als Nebenprodukt bei der Herstellung von Cyclopentanon durch Oxidation von Cyclopenten mit Distickstoffmonoxid (N₂O) bildet (siehe z.B. E. V. Starokon', K. S. Shubnikov, K. A. Dubkov, A. S. Kharitonov, G. I. Panov, Kinetics and Catalysis (2007), 48(3), 376-380). Allerdings fällt das 4-Pentenal nicht als Reinstoff an, sondern als Gemisch mit anderen Nebenprodukten aus der Cyclopentanon-Synthese.

Es wurde überraschend gefunden, dass auch derart verunreinigtes 4-Pentenal selektiv oxidiert werden kann. Die vorliegende Erfindung bietet daher den großen Vorteil, dass vor seiner entsprechenden Umsetzung keine destillative Abtrennung von reinem 4-Pentenal notwendig ist, was umso vorteilhafter ist, als die Siedepunkte von 4-Pentenal (98,5°C), Cyclopentenoxid (100,8°C) und 3-Methyl-2-butanon (94,4°C) sehr eng beieinander liegen und somit eine destillative Reinigung von 4-Pentenal nur mit großen Aufwand möglich ist.

Die vorliegende Erfindung betrifft daher ein Verfahren zur Herstellung von 4-Pentensäure mindestens umfassend den Schritt (a)
(a) Oxidation eines Gemischs (G) enthaltend 4-Pentenal, 3-Methyl-2-butanon und Cyclopentenoxid,
wobei die Oxidation gemäß Schritt (a) in Abwesenheit eines Katalysators durchgeführt wird.

Wie bereits oben erwähnt, wurde überraschend gefunden, dass auch derart verunreinigtes 4-Pentenal selektiv oxidiert werden kann und mit dem erfindungsgemäßen Verfahren 4-Pentensäure in hohen Ausbeuten und hoher Reinheit hergestellt werden kann.

Erfindungsgemäß wird gemäß Schritt (a) ein Gemisch (G) enthaltend 4-Pentenal, 3-Methyl-2-butanon und Cyclopentenoxid oxidiert, wobei 4-Pentensäure erhalten wird. Erfindungsgemäß kann das Verfahren dabei weitere Schritte, beispielsweise Reinigungsschritte umfassen.

Ein als Edukt für das erfindungsgemäße Verfahren geeignetes Gemisch (G) enthaltend 4-Pentenal, 3-Methyl-2-butanon und Cyclopentenoxid kann 4-Pentenal, 3-Methyl-2-butanon und Cyclopentenoxid in unterschiedlichen Mengenverhältnissen enthalten. Das Gemisch (G) kann dabei auch weitere Verbindungen enthalten. Geeignete Gemische (G) werden beispielsweise bei der Oxidation von Cyclopenten zu Cyclopentanon als Nebenprodukt erhalten.

Beispielsweise kann das Gemisch (G) 10 bis 90 Gew.-% 4-Pentenal enthalten, vorzugsweise 25 bis 90 Gew.-%, insbesondere 40 bis 85 Gew.-%, besonders bevorzugt 50 bis 75 Gew.-% 4-Pentenal.

Gemäß einer weiteren Ausführungsform betrifft die vorliegende Erfindung daher auch ein Verfahren zur Herstellung von 4-Pentensäure wie oben beschrieben, wobei das Gemisch (G) 10 bis 90 Gew.-% 4-Pentenal enthält.

Prinzipiell kann im Rahmen der vorliegenden Erfindung die Oxidation gemäß Schritt (a) auf jede dem Fachmann bekannte geeignete Weise erfolgen. Vorzugsweise wird jedoch als Oxidationsmittel ein sauerstoffhaltiges Gasgemisch eingesetzt, beispielsweise Luft, Sauerstoff oder ein Gemisch enthaltend Sauerstoff und ein Inertgas wie Stickstoff oder Argon.

Gemäß einer weiteren Ausführungsform betrifft die vorliegende Erfindung daher auch ein Verfahren zur Herstellung von 4-Pentensäure wie oben beschrieben, wobei für die Oxidation gemäß Schritt (a) ein sauerstoffhaltiges Gasgemisch als Oxidationsmittel eingesetzt wird.

Die Oxidation gemäß Schritt (a) kann erfindungsgemäß entweder mit oder ohne Lösungsmittel durchgeführt werden. Bevorzugt wird die Oxidation in Anwesenheit eines Lösungsmittels durchgeführt. Bei dem Lösungsmittel handelt es sich bevorzugt um eine Carbonsäure. Besonders bevorzugt werden als Lösungsmittel 4-Pentensäure, 2-Ethylhexansäure, Isononansäure, beispielsweise ein technisches Gemisch, das 3,5,5-Trimethylhexansäure als Hauptkomponente enthält, Propylheptansäure, beispielsweise ein technisches Gemisch, das 2-Propylheptansäure als Hauptkomponente enthält, oder Neodecansäure, beispielsweise ein technisches Gemisch von doppelt alpha-verzweigten Carbonsäuren mit 10 Kohlenstoffatomen, eingesetzt.

Gemäß einer weiteren Ausführungsform betrifft die vorliegende Erfindung daher auch ein Verfahren zur Herstellung von 4-Pentensäure wie oben beschrieben, wobei die Oxidation gemäß Schritt (a) in Gegenwart eines Lösungsmittels durchgeführt wird.

Insbesondere betrifft die vorliegende Erfindung auch ein Verfahren zur Herstellung von 4-Pentensäure wie oben beschrieben, wobei die Oxidation gemäß Schritt (a) in Gegenwart eines Lösungsmittels ausgewählt aus der Gruppe bestehend aus 4-Pentensäure, 2-Ethylhexansäure, Isononansäure, Propylheptansäure und Neodecansäure durchgeführt wird. Ebenso sind geeignete Mischungen aus zwei oder mehr dieser Lösungsmittel möglich.

Gemäß einer alternativen Ausführungsform betrifft die vorliegende Erfindung auch ein Verfahren zur Herstellung von 4-Pentensäure wie oben beschrieben, wobei die Oxidation gemäß Schritt (a) ohne Lösungsmittel durchgeführt wird.

Wie bereits ausgeführt kann das erfindungsgemäße Verfahren neben dem Schritt (a) weitere Schritte umfassen, beispielsweise Reinigungsschritte. So ist es erfindungsgemäß möglich, dass nach dem Schritt (a) ein weiterer Schritt anschließt, um die erhaltene 4-Pentensäure aufzukonzentrieren. Beispielsweise geeignet für die genannte Aufkonzentrierung ist unter anderem eine Destillation.

Erfindungsgemäß kann als Gemisch (G) jedes beliebige Gemisch, enthaltend 4-Pentenal, 3-Methyl-2-butanon und Cyclopentenoxid, eingesetzt werden.

Vorzugsweise wird im Rahmen der vorliegenden Erfindung als Gemisch (G) ein Gemisch eingesetzt, das als Nebenprodukt der Oxidation von Cyclopenten zu Cyclopentanon erhalten wird. Diese Verfahrensführung hat den Vorteil, dass das anfallende Nebenprodukt-Gemisch nicht aufwändig gereinigt werden muss, sondern als solches in das erfindungsgemäße Verfahren eingesetzt werden kann und so das Verfahren kostengünstig gestaltet wird.

Gemäß einer weiteren Ausführungsform betrifft die vorliegende Erfindung daher auch ein Verfahren zur Herstellung von 4-Pentensäure wie oben beschrieben, wobei das Gemisch (G) als Nebenprodukt der Oxidation von Cyclopenten zu Cyclopentanon erhalten wird.

Erfindungsgemäß ist es dabei möglich, dass das als Nebenprodukt der Oxidation von Cyclopenten zu Cyclopentanon erhaltene Gemisch enthaltend 4-Pentenal, 3-Methyl-2-butanon und Cyclopentenoxid direkt in die Oxidation gemäß Schritt (a) des erfindungsgemäßen Verfahrens eingesetzt wird. Es ist jedoch auch grundsätzlich möglich, dass ein als Nebenprodukt der Oxidation von Cyclopenten zu Cyclopentanon erhaltenes Gemisch vor Einsatz in Schritt (a) zunächst geeignet behandelt wird. Beispielsweise kann dieses Gemisch vor Schritt (a) aufkonzentriert werden und/oder der Anteil anderer, den Schritt (a) und/oder folgende Schritte störender Komponenten im Gemisch reduziert werden.

Insbesondere geeignet ist die Oxidation von Cyclopenten zu Cyclopentanon mittels Distickstoffmonoxid, da bei dieser Oxidation als Nebenprodukt Gemische anfallen, die ohne aufwändige Reinigung in die Oxidation gemäß Schritt (a) eingesetzt werden können.

Gemäß einer weiteren Ausführungsform betrifft die vorliegende Erfindung daher auch ein Verfahren zur Herstellung von 4-Pentensäure wie oben beschrieben, wobei die Oxidation von Cyclopenten zu Cyclopentanon in der Gegenwart von Distickstoffmonoxid erfolgt.

Dabei kann die Oxidation von Cyclopenten zu Cyclopentanon, insbesondere die Oxidation von Cyclopenten zu Cyclopentanon mittels Distickstoffmonoxid im Rahmen der vorliegenden Erfindung prinzipiell nach jedem geeigneten Verfahren erfolgen.

Cyclopenten kann dabei prinzipiell aus jeder beliebigen Quelle stammen. Cyclopenten kann im Rahmen der vorliegenden Erfindung als reine Substanz oder im Gemisch mit weiteren Verbindungen, insbesondere Kohlenwasserstoffen, eingesetzt werden. Dabei kann der Anteil an Kohlenwasserstoffen, beispielsweise Cyclopentan, beispielsweise im Bereich von 2 bis 80 Gew.-%, bevorzugt 20 bis 70 Gew.-%, besonders bevorzugt 40 bis 60 Gew.-%, jeweils bezogen auf das eingesetzte Gemisch, liegen.

Beispielsweise enthält das Gemisch 20 bis 98 Gew.-%, bevorzugt 30 bis 80 Gew.-%, besonders bevorzugt 40 bis 60 Gew.-%, jeweils bezogen auf das gesamte Gemisch, Cyclopenten, und 2 bis 80 Gew.-%, bevorzugt 20 bis 70 Gew.-%, besonders bevorzugt 40 bis 60 Gew.-% Cyclopentan.

Der Gehalt an anderen Komponenten im Gemisch ist beispielsweise kleiner als 15 Gew.-%, vorzugsweise kleiner als 12 Gew.-%, bevorzugt kleiner als 10 Gew.-%, insbesondere kleiner als 8 Gew.-%, besonders bevorzugt kleiner als 5 Gew.-%.

Neben den Kohlenwasserstoffen kann das Gemisch noch zu höchstens 5 Gew.-%, bevorzugt zu höchstens 2 Gew.-% mindestens eine weitere Verbindung enthalten, beispielsweise eine Verbindung ausgewählt aus der Gruppe bestehend aus Aldehyden, Ketonen, Epoxiden und Mischungen davon. Diese Verbindungen können in der Reaktionsmischung unter der Maßgabe enthalten sein, dass sie die Umsetzung von Cyclopenten mit Distickstoffmonoxid nicht stören.

Neben Cyclopenten können demgemäß mindestens ein weiterer C5-Kohlenwasserstoff, beispielsweise n-Pentan und/oder Cyclopentan, oder mindestens ein Kohlenwasserstoff mit mehr als 5 Kohlenstoffatomen, beispielsweise Cyclohexan, oder ein Gemisch aus mindestens einem weiteren C5-Kohlenwasserstoff und mindestens einem Kohlenwasserstoff mit mehr als 5 Kohlenstoffatomen im Gemisch enthalten sein.

Vorzugsweise stammt das eingesetzte Cyclopenten bzw. das Cyclopenten enthaltende Gemisch aus einem Steamcracker. In diesem Zusammenhang sind beispielsweise C5-Schnitte aus Steamcracker-Anlagen bevorzugt, die im Wesentlichen nur C5- und C6-Kohlenwasserstoffe enthalten. Kohlenwasserstoffe mit mehr als 6 Kohlenstoffatomen sind in den großtechnisch anfallenden C5-Schnitten üblicherweise nicht enthalten. Diese großtechnisch anfallenden C5-Schnitte umfassen neben Cyclopenten beispielsweise 2-Buten, Isopentan, 1-Penten, 2-Methylbuten-1, trans-2-Penten, n-Pentan, cis-2-Penten, 2-Methylbuten-2, Cyclopentan, 2,2-Dimethylbutan, 2-Methylpentan, 3-Methylpentan, n-Hexan und Benzol. Im Allgemeinen enthält ein C5-Schnitt aus einer Steamcracker-Anlage Cyclopenten im Bereich von 5 bis 60 Gew.-% und bevorzugt im Bereich von 15 bis 50 Gew.-%. Derartige Gemische werden vorteilhafterweise weiter aufgereinigt, bevor sie eingesetzt werden.

Bevorzugt wird erfindungsgemäß Cyclopenten eingesetzt, das aus einem teilhydrierten Cracker-Schnitt stammt, beispielsweise einem teilhydrierten C5-Schnitt aus einem Naphthacracker. In diesem Zusammenhang wird unter "teilhydriert" verstanden, dass der C5-Schnitt soweit hydriert wurde, bis er weitgehend frei von Dienen ist.

Dieser Schnitt wird vorzugsweise in einer ersten Destillationskolonne in einen Kopfstrom und einen Sumpfstrom aufgeteilt, wobei der Kopfstrom vorzugsweise nur noch wenig Cyclopenten, bevorzugt weniger als 10 Gew.-%, besonders bevorzugt weniger als 5 Gew.-% Cyclopenten enthält. Der Sumpfstrom enthält vorzugsweise wenig 2-Methyl-2-buten, bevorzugt weniger als 5 Gew.-%, besonders bevorzugt weniger als 1 Gew.-% und ganz besonders bevorzugt weniger als 0,5 Gew.-% 2-Methyl-2-buten. Die Destillation wird beispielsweise bei einem Druck zwischen 0,5 und 10 bar durchgeführt, bevorzugt zwischen 1 und 5 bar und besonders bevorzugt zwischen 2 und 4 bar. Die erste Kolonne weist vorzugsweise insgesamt mindestens 70 Trennstufen auf, wobei vorzugsweise mindestens 18 Trennstufen im Verstärkungsteil und mindestens 52 Trennstufen im Abtriebsteil platziert sind. Als Trennelemente können beispielsweise Böden oder Packungen verwendet werden. Bevorzugt werden Packungen eingesetzt.

Der Sumpfstrom dieser ersten Kolonne wird vorzugsweise als Feed für eine zweite Kolonne eingesetzt. In der zweiten Kolonne fällt als Kopfsrom das aufgereinigte Cyclopenten an. Das Kopfprodukt enthält vorzugsweise mindestens 80 Gew.-% Cyclopenten, bevorzugt mindestens 90 Gew.-% Cyclopenten und besonders bevorzugt mindestens 95 Gew.-% Cyclopenten. Als Hauptnebenkomponente ist im Kopfstrom Cyclopentan enthalten. Andere Nebenkomponenten, die darin enthalten sind, sind beispielsweise 2-Methyl-2-buten, 2,2-Dimethylbutan, n-Pentan, trans-2-Penten oder cis-2-Penten. Das Sumpfprodukt der zweiten Kolonne enthält vorzugsweise maximal 20 Gew.-% Cyclopenten, bevorzugt maximal 10 Gew.-% und besonders bevorzugt maximal 5 Gew.-% Cyclopenten. Dieses Sumpfprodukt enthält als Hauptkomponente Cyclopentan, vorzugsweise mindestens 50 Gew.-% Cyclopentan. Neben Cyclopentan als Hauptkomponente und Resten von Cyclopenten enthält dieser Strom beispielsweise noch 2-Methylpentan, 3-Methylpentan, Methylcyclopentan, 1-Hexen, 2,2-Dimethylbutan, n-Hexan, Benzol und 2-Methyl-2-buten als Nebenkomponenten

Es ist jedoch auch möglich, dass anstatt der zwei oben beschriebenen Kolonnen eine Trennwandkolonne verwendet wird. Eine geeignete Trennwandkolonne hat beispielsweise insgesamt 100 theoretische Trennstufen. Die Trennwand ist beispielsweise mittig platziert und überspannt beispielsweise den Bereich zwischen der 27. und der 94. Trennstufe. In diesem Fall erfolgt die Zugabe des Feeds vorzugsweise auf der Eingangsseite der Trennwand auf Höhe der Stufe 42, der Austrag vorzugsweise auf der Ausgangsseite der Trennwand auf Höhe der Stufe 54. Das Rücklaufverhältnis liegt beispielsweise im Bereich von 5 bis 7, bevorzugt von 5,5 bis 6,5 wie beispielsweise be ungefähr 6,0. Damit kann im Seitenabzug das 95%ige Cyclopentan erhalten werden. Kopf- und Sumpfstrom enthalten gemäß einer derartigen Ausführungsform vorzugsweise jeweils weniger als 2 Gew.-% Cyclopenten.

Das so erhaltene Cyclopenten kann mit Distickstoffmonoxid oxidiert werden.

Das eingesetzte Distickstoffmonoxid kann prinzipiell aus jeder beliebigen Quelle stammen. Dabei ist es möglich, reines Distickstoffmonoxid einzusetzen. Es ist jedoch ebenso möglich, Distickstoffmonoxid einzusetzen, das in reiner Form oder als Gemisch durch ein Reinigungsverfahren erhalten wurde.

Sowohl bei der Herstellung von Distickstoffmonoxid als auch bei der Verwendung von Abgasströmen fällt N₂O üblicherweise zunächst als verdünntes gasförmiges Gemisch mit anderen Komponenten an. Diese Komponenten lassen sich unterteilen in solche, die für spezielle Anwendungen störend wirken, und solche, die sich inert verhalten. Für den Einsatz als Oxidationsmittel sind als solche störend wirkenden Gase unter anderem NOx oder beispielsweise Sauerstoff (O₂) zu nennen. Der Begriff "NOx", wie er im Rahmen der vorliegenden Erfindung verstanden wird, bezeichnet sämtliche Verbindungen NₐO_{b}, wobei a gleich 1 oder 2 ist und b eine Zahl von 1 bis 6, wobei NₐO_{b} nicht N₂O ist. Statt dem Begriff "NOx" wird im Rahmen der vorliegenden Erfindung auch der Begriff "Stickoxide" verwendet. Als störende Nebenkomponenten sind auch NH₃ und organischen Säuren zu nennen.

Geeignete Reinigungsverfahren für Distickstoffmonoxid sind beispielsweise beschrieben in WO 2008/071632, oder in WO 2009/121707 und WO 2009/121706.

Ein geeignetes Verfahren zur Reinigung eines Gasgemischs enthaltend Distickstoffmonoxid ist beispielsweise ein Verfahren mindestens umfassend die Schritte
(A) Behandeln eines Gasgemischs G-0 enthaltend Distickstoffmonoxid unter Erhalt eines Gasgemischs G-A mindestens umfassend die Schritte
   (i) Absorption des Gasgemischs G-0 in einem Lösungsmittelgemisch LM-I unter Erhalt eines Abgasstroms und einer Zusammensetzung Z-A
   (ii) Desorption eines Gasgemischs G-1 aus der Zusammensetzung Z-A unter Erhalt eines Lösungsmittelgemischs LM-I'
(B) Kondensation des Gasgemischs G-A unter Erhalt einer flüssigen Zusammensetzung Z-1 enthaltend Distickstoffmonoxid und einem gasförmigen Gemisch G-K,
wobei das gasförmige Gemisch G-K in die Behandlung gemäß Schritt (A) zurückgeführt wird. Ein derartiges Verfahren und bevorzugte Ausgestaltungen sind beispielsweise in der oben zitierten WO 2009/121706 beschrieben.

Das Verfahren umfasst einen Schritt (A), umfassend die Schritte (i) und (ii). Gemäß Schritt (A) wird ein Gasgemisch G-0 enthaltend Distickstoffmonoxid unter Erhalt eines Gasgemischs G-A behandelt, wobei der Schritt (A) mindestens die Schritte (i) und (ii) umfasst. Gemäß Schritt (i) wird das Gasgemisch G-0 in einem Lösungsmittelgemisch LM-I unter Erhalt eines Abgasstroms und einer Zusammensetzung Z-A absorbiert. Gemäß Schritt (ii) wird ein Gasgemisch G-1 aus der Zusammensetzung Z-A unter Erhalt eines Lösungsmittelgemischs LM-I' desorbiert.

Bei dem Gasgemisch G-0 handelt es sich um ein Gasgemisch enthaltend Distickstoffmonoxid. Dabei kann das Gasgemisch G-0 neben Distickstoffmonoxid weitere Komponenten enthalten und grundsätzlich aus jeder beliebigen Quelle stammen.

Der Begriff "Gasgemisch", wie er im Rahmen der vorliegenden Erfindung verwendet wird, bezeichnet ein Gemisch aus zwei oder mehr Verbindungen, die sich bei Umgebungsdruck und Umgebungstemperatur im gasförmigen Zustand befinden. Bei veränderter Temperatur oder verändertem Druck kann das Gasgemisch auch in einem anderen Aggregatzustand vorliegen, beispielsweise flüssig, und wird im Rahmen der vorliegenden Erfindung weiter als Gasgemisch bezeichnet.

Im Rahmen der vorliegenden Erfindung wird die Zusammensetzung der Gasgemische oder der verflüssigten Gasgemische, wenn nicht ausdrücklich anders vermerkt, in Vol.-% angegeben. Dabei beziehen sich die Angaben auf die Zusammensetzung der Gasgemische bei Umgebungsdruck und Umgebungstemperatur.

Grundsätzlich kann die Zusammensetzung der Gemische auf jede dem Fachmann bekannte Weise bestimmt werden. Die Zusammensetzung der Gasgemische wird im Rahmen der vorliegenden Erfindung vorzugsweise gaschromatographisch bestimmt. Sie kann jedoch auch mittels UV-Spektroskopie, IR-Spektroskopie oder nasschemisch bestimmt werden.

Wird ein Gasgemisch G-0 eingesetzt, so ist dessen Gehalt an Distickstoffmonoxid im Wesentlichen beliebig, solange gewährleistet ist, dass die oben beschriebene Reinigung möglich ist.

Die N₂O-haltigen Gasgemische, die für dieses Verfahren als Gasgemisch G-0 eingesetzt werden, haben in der Regel einen N₂O-Gehalt zwischen 2 und 80 Vol.-% N₂O. Es enthält femer beispielsweise 2 bis 21 Vol.-% O₂ und bis zu 30 Vol.-% NOₓ als unerwünschte Komponenten. Ferner kann es noch in wechselnden Mengen N₂, H₂, CO₂, CO, H₂O, NH₃ enthalten, und in Spuren können auch noch organische Verbindungen enthalten sein. Beispielsweise kann das Gasgemisch G-0 auch 9 bis 13 Vol.-% N₂ und bis zu 5,5 Vol.-% NH₃ enthalten. Dabei ergibt die Summe der Komponenten des Gasgemischs G-0 100 Vol.-%.

Gemäß einer möglichen Ausführungsform des Verfahrens wird ein mindestens 3 Vol.-% Distickstoffmonoxid enthaltendes Gasgemisch G-0 eingesetzt, wobei wiederum bevorzugt Gemische mit einem Distickstoffmonoxid-Gehalt im Bereich von 4 bis 60 Vol.-%, weiter bevorzugt im Bereich von 5 bis 25 Vol.-% und insbesondere bevorzugt im Bereich von 6 bis 18 Vol.-% eingesetzt werden.

Das Gasgemisch G-0 weist gemäß dieser Ausführungsform beispielsweise einen N₂O Gehalt von 6 bis 18 Vol.-%, besonders bevorzugt beispielsweise 7 Vol.-%, 8 Vol.-%, 9 Vol.-%, 10 Vol.-%, 11 Vol.-%, 12 Vol.-%, 13 Vol.-%, 14 Vol.-%, 15 Vol.-%, 16 Vol.-% oder 17 Vol.-% auf.

Das Gasgemisch G-0 hat beispielsweise einen Gehalt an CO₂ von 0,1 bis 7,5 Vol.-%, vorzugsweise von 0,5 bis 5 Vol.-%, besonders bevorzugt 1 bis 2,5 Vol.-%. Gleichzeitig hat das Gasgemisch G-0 beispielsweise einen Gehalt an O₂ von 1 bis 10 Vol.-%, vorzugsweise von 2 bis 7,5 Vol.-%, besonders bevorzugt beispielsweise 3,0 bis 6 Vol.-%. Darüber hinaus kann das Gasgemisch G-0 noch 50 bis 95 Vol.-% N₂ enthalten, vorzugsweise 60 bis 90 Vol.-%, besonders bevorzugt 70 bis 85 Vol.-%, sowie weitere Komponenten, beispielsweise Stickoxide oder Lösungsmittelreste. NOx kann dabei beispielsweise in einer Menge von 0 bis 0,2 Vol.-% enthalten sein, vorzugsweise 0,0001 bis 0,15 Vol.-%, besonders bevorzugt 0,0005 bis 0,1 Vol.-%. Dabei ergibt die Summe der Komponenten des Gasgemischs G-0 100 Vol.-%.

Gemäß einer weiteren Ausführungsform ist das Gasgemisch G-0 enthaltend Distickstoffmonoxid mindestens ein Distickstoffmonoxid enthaltendes Abgas eines chemischen Verfahrens. Dabei sind auch Ausführungsformen umfasst, bei denen mindestens zwei Stickstoffmonoxid enthaltende Abgase einer einzigen Anlage als Gasgemisch enthaltend Distickstoffmonoxid dienen. Ebenso sind Ausführungsformen umfasst, bei denen mindestens ein Distickstoffmonoxid enthaltendes Abgas einer Anlage und mindestens ein weiteres Distickstoffmonoxid enthaltendes Abgas mindestens einer weiteren Anlage als Gasgemisch enthaltend Distickstoffmonoxid dienen.

Der Begriff "Gasgemisch enthaltend Distickstoffmonoxid" bezeichnet sowohl Ausführungsformen, in denen das genannte Abgas in unmodifizierter Form dem erfindungsgemäßen Reinigungsverfahren unterworfen wird, als auch Ausführungsformen, in denen mindestens eines der genannten Abgase einer Modifikation unterworfen wird.

Der Begriff "Modifikation", wie er in diesem Zusammenhang verwendet wird, bezeichnet jedes geeignete Verfahren, mit dem die chemische Zusammensetzung eines Gasgemisches verändert wird. Demgemäß umfasst der Begriff "Modifikation" unter anderem Ausführungsformen, in denen ein Distickstoffmonoxid enthaltendes Abgas bezüglich des Distickstoffmonoxid-Gehaltes gemäß mindestens einem geeigneten Verfahrens aufkonzentriert wird. Vorzugsweise wird das Abgas keiner Modifikation unterworfen.

Gemäß einer weiteren Ausführungsform kann die chemische Zusammensetzung eines Abgases auch durch Zusatz von reinem Distickstoffmonoxid zu dem Abgas verändert werden.

Das eingesetzte Gasgemisch G-0 enthaltend N₂O kann beispielsweise ein Abgas aus einem industriellen Verfahren sein. Vorzugsweise stammt es aus einem Abgas einer Anlage zur Herstellung von Carbonsäuren durch Oxidation von Alkoholen, Aldehyden oder Ketonen mit Salpetersäure, wie z.B. aus einer Adipinsäure-, einer Dodecandicarbonsäure- oder einer Glyoxal-Anlage, aus dem Abgas einer Salpetersäure-Anlage die die obigen Abgasströme als Edukt einsetzt, aus dem Abgas einer Anlage zur Partialoxidation von NH₃ oder aus dem Abgas einer Anlage die die darin erzeugten Gasgemische einsetzt, wie z.B. einer Hydroxylamin-Anlage.

Dabei kann auch ein Gemisch verschiedener Abgase eingesetzt werden.

Beispielsweise stammt das mindestens eine Distickstoffmonoxid enthaltende Abgas aus einer Adipinsäureanlage, einer Dodecandicarbonsäureanlage, einer Glyoxal-Anlage, einer Hydroxylaminanlage und/oder einer Salpetersäureanlage, wobei letztere wiederum bevorzugt mit mindestens einem Abgas einer Adipinsäureanlage, einer Dodecandicarbonsäureanlage oder einer Glyoxal-Anlage betrieben wird.

Beispielsweise wird der Abgasstrom einer Adipinsäureanlage eingesetzt, bei der durch Oxidation von Cyclohexanol/Cyclohexanon-Gemischen mit Salpetersäure pro Mol gebildeter Adipinsäure im Allgemeinen 0,8 bis 1,0 mol N₂O gebildet werden. Wie beispielsweise in A. K. Uriarte et al., Stud. Surf. Sci. Catal. (2000) 130 S. 743-748 beschrieben, enthalten die Abgase von Adipinsäureanlagen in unterschiedlichen Konzentrationen noch weitere Bestandteile wie unter anderem Stickstoff, Sauerstoff, Kohlendioxid, Kohlenmonoxid, Stickoxide, Wasser und flüchtige organische Verbindungen.

Bei der obenstehend erwähnten Dodecandicarbonsäureanlage handelt es sich um den im Wesentlichen identischen Anlagentyp.

Eine beispielsweise typische Zusammensetzung eines Abgases einer Adipinsäureanlage oder einer Dodecandicarbonsäureanlage ist in der folgenden Tabelle wiedergegeben:

| Komponente | Konzentrationen / Gew.-% |
|---|---|
| NO_{X} | 19 - 25 |
| N₂O | 20 - 28 |
| N₂ | 30 - 40 |
| O₂ | 7 - 10 |
| CO₂ | 2 - 3 |
| H₂O | ∼ 7 |

Der Abgasstrom einer Adipinsäureanlage oder einer Dodecandicarbonsäureanlage kann direkt in dem Reinigungsverfahren eingesetzt werden.

Gemäß einer weiteren Ausführungsform wird der Abgasstrom einer Salpetersäureanlage eingesetzt, die ganz oder teilweise mit Distickstoffmonoxid und Stickoxide enthaltenden Abgasen aus anderen Verfahren gespeist wird. In derartigen Salpetersäureanlagen werden Stickoxide adsorbiert und zum größten Teil zu Salpetersäure umgesetzt, während Distickstoffmonoxid nicht umgesetzt wird. Beispielsweise kann eine derartige Salpetersäureanlage durch Stickoxide, die durch gezielte Verbrennung von Ammoniak hergestellt werden, und durch Abgase einer Adipinsäureanlage und/oder durch Abgase einer Dodecandicarbonsäureanlage und/oder durch Abgase einer Glyoxal-Anlage gespeist werden. Ebenso ist es möglich, eine derartige Salpetersäureanlage allein durch Abgase einer Adipinsäureanlage und/oder durch Abgase einer Dodecandicarbonsäureanlage und/oder durch Abgase einer Glyoxal-Anlage zu speisen.

Die Abgase von derartigen Salpetersäureanlagen enthalten grundsätzlich in unterschiedlichen Konzentrationen noch weitere Bestandteile wie unter anderem Stickstoff, Sauerstoff, Kohlendioxid, Kohlenmonoxid, Stickoxide, Wasser und flüchtige organische Verbindungen.

Eine beispielsweise typische Zusammensetzung eines Abgases einer derartigen Salpetersäureanlage ist in der folgenden Tabelle wiedergegeben:

| Komponente | Konzentrationen / Gew.-% |
|---|---|
| NO_{X} | < 0,1 |
| N₂O | 4-36 |
| N₂ | 57 - 86 |
| O₂ | 3-9 |
| CO₂ | 1 - 4 |
| H₂O | ∼ 0,6 |

Der Abgasstrom einer Salpetersäureanlage kann direkt in einem derartigen Reinigungsverfahren eingesetzt werden.

Gemäß einer weiteren Ausführungsform wird der Abgasstrom einer Hydroxylamin-anlage eingesetzt, bei der beispielsweise zunächst Ammoniak mit Luft oder Sauerstoff zu NO oxidiert wird, wobei kleine Mengen an Distickstoffmonoxid als Nebenprodukt gebildet werden. Das NO wird anschließend mit Wasserstoff zu Hydroxylamin hydriert. Nachdem Distickstoffmonoxid unter den Hydrierbedingungen inert ist, reichert es sich im Wasserstoffkreis an. In bevorzugten Verfahrensführungen enthält der Purge-Strom einer Hydroxylaminanlage Distickstoffmonoxid im Bereich von 9 bis 13 Vol.-% in Wasserstoff. Dieser Purge-Strom kann als solcher zur erfindungsgemäßen Reinigung eingesetzt werden. Ebenso ist es möglich, diesen Strom bezüglich des Distickstoffmonoxid-Gehaltes, wie oben beschrieben, geeignet aufzukonzentrieren.

Ebenso kann Distickstoffmonoxid zum Einsatz im Reinigungsverfahren gezielt hergestellt werden. Bevorzugt wird dabei unter anderem die Herstellung über die thermische Zersetzung von NH₄NO₃, wie dies beispielsweise in US 3,656,899 beschrieben ist. Ebenfalls bevorzugt wird weiter die Herstellung über die katalytische Oxidation von Ammoniak, wie dies beispielsweise in US 5,849,257 oder in WO 98/25698 beschrieben ist.

Bei der Absorption gemäß Schritt (i) wird das Gasgemisch G-0 in einem Lösungsmittelgemisch LM-I absorbiert. Dabei ist es grundsätzlich jede dem Fachmann bekannte Methode zur Absorption einzusetzen. Dabei werden ein Abgasstrom und eine Zusammensetzung Z-A erhalten. Die Zusammensetzung Z-A wird dann in Schritt (ii) weiter behandelt. Dabei wird das Gasgemisch G-1 aus der Zusammensetzung Z-A unter Erhalt eines Lösungsmittelgemischs LM-I' desorbiert.

Das Gasgemisch G-1 enthält dabei mindestens Distickstoffmonoxid und kann weitere Komponenten enthalten.

Dabei kann als Lösungsmittelgemisch LM-I jedes dem Fachmann bekannte geeignete Lösungsmittelgemisch eingesetzt werden, solange gewährleistet ist, dass das Gasgemisch G-0, insbesondere Distickstoffmonoxid, zumindest teilweise absorbiert wird.

Gemäß Schritt (A) wird ein Gasgemisch G-A erhalten, das Distickstoffmonoxid enthält. Das Gasgemisch G-A kann darüber hinaus weitere Komponenten enthalten. Sofern der Schritt (A) nach dem Schritt (ii) keine weiteren Schritte umfasst, ist die Zusammensetzung des Gasgemischs G-1 identisch mit der des Gasgemischs G-A.

Gemäß Schritt (B) wird das aus Schritt (A) erhaltene Gasgemisch G-A unter Erhalt einer flüssigen Zusammensetzung Z-1 enthaltend Distickstoffmonoxid und einem gasförmigen Gemisch G-K zumindest teilweise kondensiert. Dabei enthält die flüssige Zusammensetzung Z-A Distickstoffmonoxid und kann weitere Komponenten enthalten. Das gasförmige Gemisch G-K enthält vorzugsweise nur geringe Mengen an Distickstoffmonoxid. Gemäß diesem Verfahren wird nach der Kondensation gemäß Schritt (B) das gasförmige Gemisch G-K in die Behandlung gemäß Schritt (A) zurückgeführt.

Dabei kann das Verfahren weitere Schritte umfassen. So ist es beispielsweise möglich, dass zwischen den Schritten (A) und (B) weitere Schritte umfasst sind.

Ebenso kann der Schritt (A) auch weitere Schritte umfassen. Dabei ist es insbesondere möglich, dass der Schritt (A) eine weitere Absorption des Gasgemischs G-1 in einem geeigneten Lösungsmittelgemisch und eine weitere Desorption umfasst:
(iii) Absorption des Gasgemischs G-1 in einem Lösungsmittelgemisch LM-II unter Erhalt eines Abgasstroms und einer Zusammensetzung Z-B
(iv) Desorption eines Gasgemischs G-2 aus der Zusammensetzung Z-B unter Erhalt eines Lösungsmittelgemischs LM-II'.

Dabei kann als Lösungsmittelgemisch LM-II jedes dem Fachmann bekannte geeignete Lösungsmittelgemisch eingesetzt werden, solange gewährleistet ist, dass das Gasgemisch G-1, insbesondere Distickstoffmonoxid, zumindest teilweise absorbiert wird.

Sofern der Schritt (A) nach dem Schritt (iv) keine weiteren Schritte umfasst, ist die Zusammensetzung des Gasgemischs G-2 identisch mit der des Gasgemischs G-A.

Ebenso ist es möglich, dass der Schritt (A) neben den Schritten (i) und (ii) oder neben den Schritten (i), (ii), (iii) und (iv) weitere Schritte umfasst, wie beispielsweise auch weitere Absorptionen und Desorptionen.

Wie zuvor beschrieben, wird das gemäß Schritt (B) erhaltene gasförmige Gemisch G-K in den Schritt (A) des Verfahrens zurückgeführt. Dabei wird das gasförmige Gemisch G-K mit einem anderen Gasgemisch gemischt. Vorzugsweise wird dabei das gasförmige Gemisch G-K derart in den Schritt (A) zurückgeführt, dass eine Gewinnung des gegebenenfalls im gasförmigen Gemisch G-K enthaltenen Distickstoffmonoxids möglich ist. Daher ist es bevorzugt, dass das gasförmige Gemisch G-K mit einem Gasgemisch gemischt wird, das einer Absorption zugeführt wird, insbesondere dem Gasgemisch G-0 oder dem Gasgemisch G-1. Somit ist es im Rahmen der vorliegenden Erfindung bevorzugt, das gasförmige Gemisch G-K in Schritt (i) oder in Schritt (iii) des Schritts (A) zurückzuführen.

Dabei kann als Lösungsmittelgemisch LM-I und oder LM-II jedes dem Fachmann bekannte geeignete Lösungsmittelgemisch eingesetzt werden, solange gewährleistet ist, dass insbesondere Distickstoffmonoxid absorbiert wird.

Geeignete Lösungsmittelgemische LM-I und LM-II für die Absorption gemäß Schritt (i) oder (iii) sind solche, die für N₂O und vorzugsweise auch CO₂ als inerte Komponente eine bessere Löslichkeit aufweisen als für die unerwünschten Komponenten des eintretenden Eduktgases G-0.

So können als Lösungsmittelgemisch LM-I und/oder LM-II organische Lösungsmittel oder wässrige Lösungsmittelgemische eingesetzt werden. Als organische Lösungsmittel können alle Lösungsmittel eingesetzt werden, bei denen das Verhältnis zwischen N₂O-Löslichkeit (in mol/mol Lösungsmittel) und der Löslichkeit der unerwünschten Nebenkomponenten unter den im Absorber herrschenden Bedingungen (dieses Verhältnis wird im Folgenden "gamma" genannt) mindestens 5 beträgt. Dies Verhältnis kann für jede einzelne im Gasgemisch enthaltene Komponente bestimmt werden. Bevorzugte organische Lösungsmittel weisen beispielsweise bei 30 °C einen Wert gamma(O₂) von 6 bis 30, bevorzugt von 9 bis 25 und einen Wert gamma(N₂) von größer 10, bevorzugt von größer als 15, insbesondere von größer als 20 auf.

Beispiele für geeignete organische Lösungsmittel sind beispielsweise aliphatische Kohlenwasserstoffe, bevorzugt mit mindestens 5 C-Atomen, weiter bevorzugt mit mindestens 8 C-Atomen, substituierte oder unsubstituierte aromatische Kohlenwasserstoffe, Ester, Ether, Amide, Lactone, Lactame, Nitrile, Alkylhalogenide, Olefine oder Mischungen dieser Lösungsmittel.

Ganz besonders bevorzugt sind organische Lösungsmittel, die einen Siedepunkt bei Normaldruck von mindestens 100°C aufweisen, da dadurch die Lösungsmittelverluste sowohl im Abgasstrom des Absorbers wie auch des Desorbers reduziert werden.

Darüber hinaus weisen geeignete Lösungsmittel gleichzeitig eine gute Löslichkeit für Distickstoffmonoxid auf. Die Löslichkeit wird über das Verhältnis zwischen dem Partialdruck von N₂O in der Gasphase und dem Molanteil von N₂O in der Flüssigphase (Henry-Koeffizient, H_{N2O}) angegeben, d.h. ein kleiner Wert bedeutet eine hohe Löslichkeit von Distickstoffmonoxid im Lösungsmittel. Vorzugsweise ist dies Verhältnis für ein insbesondere in dem ersten Schritt eingesetztes organisches Lösungsmittel bei 30°C kleiner als 1000, weiter bevorzugt kleiner als 750, besonders bevorzugt kleiner als 500, insbesondere kleiner als 150.

Geeignete organische Lösungsmittel sind unter anderem N-Methylpyrrolidon, Dimethylformamid, Dimethylsulfoxid, Propylencarbonat, Sulfolan, N,N-Dimethylacetamid oder Cyclopentan. Besonders bevorzugt sind im Rahmen der vorliegenden Erfindung beispielsweise Toluol, Nitrobenzol, 1,2-Dichlorbenzol, Tetradecan, beispielsweise ein technisches Gemisch aus gesättigten Kohlenwasserstoffen mit überwiegend 14 Kohlenstoffatomen, und Phthalsäuredimethylester.

Ebenso ist es möglich, als Lösungsmittelgemisch LM-I und/oder LM-II wässrige Lösungsmittelgemische einzusetzen. Dabei gelten für die Eignung der Lösungsmittelgemische für das Verfahren grundsätzlich die obigen Ausführungen. Insbesondere können als Lösungsmittelgemisch LM-I und/oder LM-II Lösungsmittelgemische mindestens enthaltend 50 Gew.-% Wasser bezogen auf das gesamte Lösungsmittelgemisch, eingesetzt werden. Dabei ist es im Rahmen der vorliegenden Erfindung auch möglich, dass der pH-Wert des eingesetzten Lösungsmittelgemischs in einem bestimmten Bereich eingestellt wird. Ein geeigneter pH-Wert für ein wässriges Lösungsmittelgemisch liegt erfindungsgemäß beispielsweise im Bereich von 2,0 bis 8,0. Dabei ist es erfindungsgemäß auch möglich, dass der pH-Wert der in den einzelnen Absorptionsschritten eingesetzten Lösungsmittelgemische LM-I oder LM-II variiert.

Dabei wird der pH-Wert mit einer kommerziell erhältlichen Glaselektrode gemessen, die vorher gegen Puffer mit bekanntem pH-Wert kalibriert wurde. Alle Angaben von pH-Werten beziehen sich auf eine Messung mit einer kalibrierten und temperaturkompensierten Glaselektrode. Falls die Kalibriertemperatur von der Messtemperatur abweicht wird eine Temperaturkompensierung verwendet. Diese Definition und dieses Vorgehen entspricht der derzeit gültigen IUPAC Empfehlung (R.P.Buck et al., Pure Appl. Chem. (2002) 74(11), S. 2169-2200 und dort insbesondere Abschnitt 11).

Wasser weist eine hohe Selektivität für die gewünschten Komponenten, insbesondere Distickstoffmonoxid und Kohlendioxid, auf. Gleichzeitig ist die absolute Löslichkeit von Distickstoffmonoxid in Wasser ausreichend, um eine weitere Aufkonzentrierung zu erreichen. Dabei hat Wasser als Lösungsmittel den Vorteil, dass auch unter Druck in Gegenwart von konzentriertem Distickstoffmonoxid keine sicherheitstechnischen Probleme auftreten. Gleichzeitig kann keine Kontaminierung des Gasgemischs G-2 mit einem organischen Lösungsmittel auftreten, die zusätzliche Reinigungsschritte nötig machen würden.

Somit können sowohl das Lösungsmittelgemisch LM-I als auch LM-II ein organisches Lösungsmittelgemisch oder ein wässriges Lösungsmittelgemisch sein. Es ist möglich, dass als Lösungsmittelgemisch LM-I ein organisches Lösungsmittel eingesetzt wird und als Lösungsmittelgemisch LM-II ein wässriges Lösungsmittelgemisch. Ebenso ist es möglich, dass als Lösungsmittelgemisch LM-I ein wässriges Lösungsmittelgemisch eingesetzt wird und als Lösungsmittelgemisch LM-II ein organisches Lösungsmittel. Vorzugsweise sind sowohl das Lösungsmittelgemisch LM-I als auch das Lösungsmittelgemisch LM-II ein wässriges Lösungsmittelgemisch.

Weiter ist es bevorzugt, dass sofern als Lösungsmittelgemisch LM-I und/oder LM-II ein wässriges Lösungsmittelgemisch eingesetzt wird, der pH-Wert des wässrigen Lösungsmittelgemischs in einem bestimmten Bereich eingestellt wird.

Beispielsweise kann der pH-Wert des wässrigen Lösungsmittelgemischs bei der Absorption vorzugsweise im Bereich von 3,5 bis 8,0 liegen. Bei diesem pH-Wert erfolgt erfindungsgemäß eine gute Absorption von Distickstoffmonoxid und Kohlenstoffdioxid im Lösungsmittelgemisch, wobei andere Gase, die im Gasgemisch G-0 enthalten sein können, nicht oder wenig absorbiert werden. Bevorzugt liegt der pH-Wert in einem Bereich von 5,0 bis 7,5 besonders bevorzugt in einem Bereich von 6,0 bis 7,0.

Dabei wird der pH-Wert vor oder während des Inkontaktbringens des Gasgemischs mit dem wässrigen Lösungsmittelgemisch gemessen und dann beispielsweise der pH-Wert durch geeignete Maßnahmen eingestellt. Ebenso ist es möglich, dass keine Maßnahmen nötig sind, um den pH-Wert einzustellen.

Grundsätzlich kann der pH-Wert durch alle dem Fachmann bekannten Maßnahmen eingestellt werden. Geeignete Maßnahmen zum Einstellen des pH-Werts sind beispielsweise Zugabe einer Säure oder Lauge oder Zugabe von weiterem Lösungsmittel.

Beispielsweise wird der pH-Wert des wässrigen Lösungsmittelgemischs vor oder nach der Absorption gemessen und der pH-Wert im genannten Bereich durch geeignete Maßnahmen eingestellt. Die Messung des pH-Werts kann dabei kontinuierlich oder diskontinuierlich erfolgen.

Sofern der pH-Wert des Lösungsmittelgemischs LM-I und des Lösungsmittelgemischs LM-II eingestellt werden, können der pH-Wert des Lösungsmittelgemischs LM-I und des Lösungsmittelgemischs LM-II unabhängig voneinander eingestellt werden. So ist es auch möglich, dass nur der pH-Wert des Lösungsmittelgemischs LM-I oder des Lösungsmittelgemischs LM-II eingestellt werden. Der pH-Wert des Lösungsmittelgemischs LM-I und des Lösungsmittelgemischs LM-II können jedoch auch im gleichen Bereich eingestellt werden.

Unter einem wässrigen Lösungsmittelgemisch wird dabei ein Lösungsmittelgemisch verstanden, mindestens enthaltend 50 Gew.-% Wasser beispielsweise 50 bis 100 Gew.-% Wasser, vorzugsweise mindestens 60 Gew.-% Wasser, insbesondere mindestens 70 Gew.-% Wasser, besonders bevorzugt mindestens 80 Gew.-% Wasser, beispielsweise mindestens 90 Gew.-% Wasser. Vorzugsweise enthält das wässrige Lösungsmittelgemisch mindestens 90 Gew.-% Wasser, jeweils bezogen auf das gesamte wässrige Lösungsmittelgemisch.

Dabei kann das wässrige Lösungsmittelgemisch neben Wasser auch andere polare mit Wasser mischbare Lösungsmittel enthalten, beispielsweise Glykole. Darüber hinaus kann das wässrigen Lösungsmittelgemisch neben Wasser auch gelöste Salze enthalten, beispielsweise Salze der Alkali- oder Erdalkalimetalle, insbesondere Hydroxide, Hydrogencarbonate, Carbonate, Nitrate, Nitrite, Sulfate, Hydrogenphosphate oder Phosphate.

Beispielsweise ist der Gehalt an Salzen im wässrigen Lösungsmittelgemisch kleiner als 5 Gew.-%, bevorzugt kleiner als 2,5 Gew.-%, besonders bevorzugt kleiner als 2,0 Gew.-%. Der Gehalt an Salzen im wässrigen Lösungsmittelgemisch beträgt beispielsweise 0,0001 bis 5 Gew.-%, bevorzugt 0,001 bis 2,5 Gew.-%, insbesondere 0,01 bis 2,0 Gew.-%.

Dabei wird der Gehalt an Salzen im wässrigen Lösungsmittelgemisch vorzugsweise dadurch kontrolliert, dass kontinuierlich oder diskontinuierlich einen Teil des mit Salzen beladenen Lösungsmittelgemischs durch eine entsprechend angepasste Menge an frischem Lösungsmittelgemisch ersetzt wird.

Gemäß Schritt (i) erfolgt erfindungsgemäß eine zumindest teilweise Absorption des Gasgemischs G-0 in einem Lösungsmittelgemisch LM-I, wobei eine Zusammensetzung Z-A und ein an den absorbierten Gasen verarmter Abgasstrom erhalten wird.

Dabei wird unter einem verarmten Abgasstrom ein Gasstrom verstanden, der die nicht bei der Absorption im Lösungsmittelgemisch LM-I oder LM-II absorbierten Gase enthält.

Die Zusammensetzung Z-A umfasst das Lösungsmittelgemisch LM-I und die darin absorbierten Gase.

Sofern als Lösungsmittelgemisch LM-I Wasser eingesetzt wird, enthält die Zusammensetzung Z-A beispielsweise 90,0 bis 99,9999 Gew.-% Wasser, insbesondere 95,0 bis 99,999 Gew.-%, bevorzugt 98,0 bis 99,99 Gew.-% Wasser; beispielsweise 0,01 bis 0,25 Gew.-% Distickstoffmonoxid, insbesondere 0,05 bis 0,2 Gew.-%, bevorzugt 0,1 bis 0,15 Gew.-% Distickstoffmonoxid; beispielsweise 0,0001 bis 0,1 Gew.-% Kohlenstoffdioxid, insbesondere 0,001 bis 0,05 Gew.-% Kohlenstoffdioxid; beispielsweise 0,0001 bis 0,1 Gew.-% Stickstoff, insbesondere 0,001 bis 0,05 Gew.-% Stickstoff; beispielsweise 0,05 bis 1,5 Gew.-% Natriumnitrit, insbesondere 0,1 bis 1,0 Gew.-%, bevorzugt 0,25 bis 0,75 Gew.-% Natriumnitrit; beispielsweise 0,05 bis 1,5 Gew.-% Natriumnitrat, insbesondere 0,1 bis 1,0 Gew.-%, bevorzugt 0,25 bis 0,75 Gew.-% Natriumnitrat; ; beispielsweise 0,0001 bis 0,1 Gew.-% Natriumhydrogencarbonat, insbesondere 0,001 bis 0,05 Gew.-% Natriumhydrogencarbonat; sowie Spuren von Sauerstoff und Argon. Dabei ergibt die Summe der Komponenten der Zusammensetzung (A) 100 Gew.-%.

Dabei enthält der verarmte Abgasstrom beispielsweise 0,1 bis 2,0 Vol.-% Argon, insbesondere 0,25 bis 1,5 Vol.-%, bevorzugt 0,5 bis 1,0 Vol.-% Argon; beispielsweise 1,0 bis 10 Vol.-% Sauerstoff, insbesondere 2,5 bis 7,5 Vol.-%, bevorzugt 4,0 bis 6,0 Vol.-% Sauerstoff; beispielsweise 1,0 bis 10 Vol.-% Distickstoffmonoxid, insbesondere 2,5 bis 7,5 Vol.-%, bevorzugt 4,0 bis 6,0 Vol.-% Distickstoffmonoxid; beispielsweise 70 bis 99,9 Vol.-% Stickstoff, insbesondere 75 bis 95 Vol.-%, bevorzugt 80 bis 90 Vol.-% Stickstoff; beispielsweise 0,01 bis 0,5 Vol.-% Kohlenstoffmonoxid, insbesondere 0,05 bis 0,25 Vol.-%, bevorzugt 0,08 bis 0,1 Vol.-% Kohlenstoffmonoxid; beispielsweise 0,1 bis 1,5 Vol.-% Kohlenstoffdioxid, insbesondere 0,25 bis 1,0 Vol.-%, bevorzugt 0,5 bis 0,75 Vol.-% Kohlenstoffdioxid; beispielsweise 0,1 bis 1,5 Vol.-% Wasser, insbesondere 0,25 bis 1,0 Vol.-%, bevorzugt 0,5 bis 0,75 Vol.-% Wasser. Dabei ergibt die Summe der Komponenten des Abgasstroms 100 Vol.-%.

Vorzugsweise wird Schritt (i) des Verfahrens kontinuierlich durchgeführt. Das heißt, dass kontinuierlich das Lösungsmittelgemisch LM-I und das Gasgemisch G-0 in Kontakt gebracht werden, wobei sich kontinuierlich die Zusammensetzung Z-A und der verarmte Abgasstrom bilden.

Dabei werden bei der Absorption gemäß Schritt (i) vorzugsweise Distickstoffmonoxid und Kohlendioxid absorbiert. So können beispielsweise auch Stickstoff, Sauerstoff und Argon absorbiert werden. Auch Stickoxide NOₓ werden gemäß Schritt (i) absorbiert.

Das Verfahren umfasst weiter einen Schritt (ii), bei dem ein Gasgemisch G-1 aus der Zusammensetzung Z-A unter Erhalt eines Lösungsmittelgemischs LM-I' desorbiert wird.

Dabei werden gemäß Schritt (ii) vorzugsweise Distickstoffmonoxid und Kohlenstoffdioxid aus der Zusammensetzung Z-A desorbiert.

Das Lösungsmittelgemisch LM-I' enthält neben dem eingesetzten Lösungsmittelgemisch LM-I noch nicht desorbierte Gase und Folgeprodukte.

Beispielsweise für den Fall, dass als Lösungsmittelgemisch LM-I mit einem bestimmten eingestellten pH-Wert eingesetzt wird und der pH-Wert durch Zugabe einer Lauge, insbesondere Natronlauge, eingestellt wird, enthält das Lösungsmittelgemisch LM-I' beispielsweise 90,0 bis 99,9999 Gew.-% Wasser, insbesondere 95,0 bis 99,999 Gew.-%, bevorzugt 98,0 bis 99,99 Gew.-% Wasser; beispielsweise 0,001 bis 0,1 Gew.-% Distickstoffmonoxid, beispielsweise 0,05 bis 1,5 Gew.-% Natriumnitrit, insbesondere 0,1 bis 1,0 Gew.-%, bevorzugt 0,25 bis 0,75 Gew.-% Natriumnitrit; beispielsweise 0,05 bis 1,5 Gew.-% Natriumnitrat, insbesondere 0,1 bis 1,0 Gew.-%, bevorzugt 0,25 bis 0,75 Gew.-% Natriumnitrat; ; beispielsweise 0,0001 bis 0,1 Gew.-% Natriumhydrogencarbonat, insbesondere 0,001 bis 0,05 Gew.-% Natriumhydrogencarbonat. Das Lösungsmittelgemisch LM-I' kann darüber hinaus auch weitere Verbindungen enthalten. Dabei ergibt die Summe der Komponenten des Lösungsmittelgemischs LM-I' 100 Gew.-%.

Dabei hat das Gasgemisch G-1 beispielsweise einen Gehalt an N₂O von 40 bis 80 Vol.-%, vorzugsweise von 45 bis 75 Vol.-%, insbesondere von 50 bis 65 Vol.-%, besonders bevorzugt beispielsweise 51 Vol.-%, 52 Vol.-%, 53 Vol.-% 54 Vol.-%, 55 Vol.-%, 56 Vol.-%, 57 Vol.-%, 58 Vol.-%, 59 Vol.-%, 60 Vol.-%, 61 Vol.-%, 62 Vol.-%, 63 Vol.-%, 64 Vol.-% oder 65 Vol.-%.

Das Gasgemisch G-1 hat beispielsweise einen Gehalt an CO₂ von 5 bis 15 Vol.-%, vorzugsweise von 6 bis 12 Vol.-%, besonders bevorzugt beispielsweise 7 Vol.-%, 9 Vol.-%, 10 Vol.-% oder 11 Vol.-%. Gleichzeitig hat das Gasgemisch G-1 beispielsweise einen Gehalt an O₂ von 1,0 bis 4,0 Vol.-%, vorzugsweise von 1,5 bis 3,5 Vol.-%, besonders bevorzugt 2,5 bis 3.1 Vol.-%, beispielsweise 2,6 Vol.-%, 2,7 Vol.-%, 2,8 Vol.-%, 2,9 Vol.-% oder 3,0 Vol.-%. Darüber hinaus kann das Gasgemisch G-1 noch 20 bis 40 Vol.-% N₂ enthalten, vorzugsweise 20 bis 35 Vol.-%, sowie weitere Komponenten, beispielsweise Stickoxide. NOₓ kann dabei beispielsweise in einer Menge von 0 bis 0,1 Vol.-% enthalten sein, vorzugsweise 0,0001 bis 0,01 Vol.-%, besonders bevorzugt 0,0002 bis 0,05 Vol.-%. Dabei ergibt die Summe der Komponenten des Gasgemischs G-1 100 Vol.-%. Das Gasgemisch G-1 kann darüber hinaus noch 0 bis 10 Vol.-% Wasser enthalten, insbesondere 2 bis 8 Vol.-%, bevorzugt 4 bis 6 Vol.-% Wasser.

Das Verfahren kann weiter einen Schritt (iii) und einen Schritt (iv) aufweisen. Bei der Absorption gemäß Schritt (iii) erfolgt eine Absorption in einem Lösungsmittelgemisch LM-II wobei eine Zusammensetzung Z-B und ein an den absorbierten Gasen verarmter Abgasstrom erhalten wird. Die Zusammensetzung Z-B umfasst das Lösungsmittelgemisch LM-II und die darin absorbierten Gase.

Sofern als Lösungsmittelgemisch LM-II Wasser eingesetzt wird, enthält die Zusammensetzung Z-B beispielsweise 90,0 bis 99,9999 Gew.-% Wasser, insbesondere 95,0 bis 99,999 Gew.-%, bevorzugt 98,0 bis 99,99 Gew.-% Wasser; beispielsweise 0,01 bis 2,5 Gew.-% Distickstoffmonoxid, insbesondere 0,1 bis 1,5 Gew.-%, bevorzugt 0,5 bis 1,0 Gew.-% Distickstoffmonoxid; beispielsweise 0,001 bis 0,5 Gew.-% Kohlenstoffdioxid, insbesondere 0,01 bis 0,25 Gew.-% Kohlenstoffdioxid; beispielsweise 0,0001 bis 0,1 Gew.-% Stickstoff, insbesondere 0,001 bis 0,05 Gew.-% Stickstoff; sowie Spuren von Sauerstoff und Argon. Dabei ergibt die Summe der Komponenten der Zusammensetzung Z-B 100 Gew.-%.

Vorzugsweise wird Schritt (iii) kontinuierlich durchgeführt. Das heißt, dass kontinuierlich das Lösungsmittelgemisch LM-II und das Gasgemisch G-1 in Kontakt gebracht werden, wobei sich kontinuierlich die Zusammensetzung Z-B und der verarmte Abgasstrom bilden. Vom eintretenden Gasstrom werden in Schritt (iii) vorzugsweise 60 bis 80 % absorbiert.

Das Verfahren umfasst vorzugsweise weiter einen Schritt (iv), bei dem ein Gasgemisch G-2 aus der Zusammensetzung Z-B unter Erhalt eines Lösungsmittelgemischs LM-II' desorbiert wird. Dabei werden gemäß Schritt (iv) vorzugsweise Distickstoffmonoxid und Kohlenstoffdioxid aus der Zusammensetzung Z-B desorbiert. Das Lösungsmittelgemisch LM-II' enthält neben dem eingesetzten Lösungsmittelgemisch LM-II noch nicht desorbierte Gase und Folgeprodukte.

Das erhaltene Gasgemisch G-2 enthält mindestens 50 Vol.-% N₂O, besonders bevorzugt mindestens 60 Vol.-% N₂O und ganz besonders bevorzugt mindestens 75 Vol.-% N₂O. Üblicherweise enthält das Gasgemisch G-2 bis zu 99 Vol.-% N₂O, insbesondere bis zu 97 Vol.-% N₂O, beispielsweise bis zu 96 Vol.-% N₂O, bis zu 95 Vol.-% N₂O, bis zu 94 Vol.-% N₂O, bis zu 93 Vol.-% N₂O, bis zu 92 Vol.-% N₂O, bis zu 91 Vol.-% N₂O, bis zu 90 Vol.-% N₂O oder auch bis zu 85 Vol.-% N₂O.

Dabei hat das Gasgemisch G-2 beispielsweise einen Gehalt an N₂O von 60 bis 95 Vol.-%, vorzugsweise von 70 bis 90 Vol.-%, insbesondere von 75 bis 85 Vol.-%, besonders bevorzugt beispielsweise 76 Vol.-%, 77 Vol.-%, 78 Vol.-%, 79 Vol.-%, 80 Vol.-%, 81 Vol.-%, 82 Vol.-%, 83 Vol.-%, 84 Vol.-% oder 85 Vol.-%.

Das Gasgemisch G-2 hat beispielsweise einen Gehalt an CO₂ von 1 bis 20 Vol.-%, vorzugsweise von 5 bis 15 Vol.-%, besonders bevorzugt beispielsweise 6 Vol.-%, 7 Vol.-%, 8 Vol.-%, 9 Vol.-%, 10 Vol.-%, 11 Vol.-%, 12 Vol.-%, 13 Vol.-% oder 14 Vol.-%. Gleichzeitig hat das Gasgemisch G-2 beispielsweise einen Gehalt an O₂ von 0,01 bis 5,0 Vol.-%, vorzugsweise von 0,1 bis 2,5 Vol.-%, besonders bevorzugt beispielsweise 0,2 bis 1,0 Vol.-%. Darüber hinaus kann das Gasgemisch G-2 noch 0,1 bis 10 Vol.-% N₂ enthalten, vorzugsweise 0,5 bis 5 Vol.-%, sowie weitere Komponenten, beispielsweise Stickoxide oder Lösungsmittelreste. Gleichzeitig enthält das Gasgemisch G-2 weniger als 1 Vol.-% O₂, insbesondere weniger als 0,5 Vol.-% O₂, weniger als 0,5 Vol.-% NOₓ. NOₓ kann dabei beispielsweise in einer Menge von 0 bis 0,1 Vol.-% enthalten sein, vorzugsweise 0,0001 bis 0,01 Vol.-%, besonders bevorzugt 0,0002 bis 0,02 Vol.-%. Dabei ergibt die Summe der Komponenten des Gasgemischs G-2 100 Vol.-%.

Sofern der Schritt (A) nach dem Schritt (iv) keine weiteren Schritte umfasst, entspricht die Zusammensetzung des Gasgemischs G-A der Zusammensetzung des Gasgemischs G-2.

Dabei können sowohl das Lösungsmittelgemisch LM-I' als auch das Lösungsmittelgemisch LM-II' zumindest teilweise in das Verfahren zurückgeführt werden. Dabei ist es möglich, dass das Lösungsmittelgemisch LM-I' und/oder das Lösungsmittelgemisch LM-II' zumindest teilweise als Lösungsmittelgemisch LM-I oder LM-II in das Verfahren zurückgeführt werden. Dabei kann das Lösungsmittelgemisch LM-I' und/oder das Lösungsmittelgemisch LM-II' insbesondere behandelt werden, bevor es als Lösungsmittelgemisch LM-I oder LM-II wieder in das Verfahren eingesetzt wird.

Insbesondere ist es auch möglich, dass nur ein Teil des Lösungsmittelgemischs LM-I' und/oder LM-II' wieder in das Verfahren eingesetzt wird und beispielsweise mit Wasser oder einem anderen Lösungsmittel versetzt wird, um dann als Lösungsmittelgemisch LM-I und/oder LM-II wieder in das Verfahren eingesetzt zu werden.

Die Absorption gemäß Schritt (i) bzw. (iii) des beschriebenen Verfahrens kann grundsätzlich nach allen dem Fachmann bekannten Verfahren erfolgen. Insbesondere kann die Absorption im Lösungsmittelgemisch durch Erhöhung des Drucks des Eduktgases oder durch Absenkung der Temperatur des Lösungsmittelgemischs oder durch eine Kombination der genannten Maßnahmen herbeigeführt werden.

Vorzugsweise wird bei Schritt (i) bzw. (iii) des Verfahrens zunächst das Gasgemisch komprimiert, beispielsweise auf einen Druck von 10 bis 35 bar, bevorzugt von 13 bis 30 bar, vorzugsweise von 14 bis 25 bar. Anschließend wird das komprimierte Gasgemisch vorzugsweise bei diesem Druck mit dem Lösungsmittelgemisch LM-I gemäß Schritt (i) oder in dem Lösungsmittelgemisch LM-II gemäß Schritt (iii) in Kontakt gebracht.

Die Absorption gemäß Schritt (i) und Schritt (iii) erfolgt in Einrichtungen (Absorber), in denen eine Gas-Flüssig-Phasengrenzfläche erzeugt wird, über die ein Stoff- und Wärmeübergang zwischen den Phasen ermöglicht wird, und die bei Bedarf mit internen oder externen Einrichtungen zur Wärmezufuhr und/oder Wärmeabfuhr versehen sind.

Die Führung der Phasen im Absorber kann im Gleichstrom, im Gegenstrom oder einer Kombination der genannten erfolgen.

Die Absorption kann ein- oder mehrstufig durchgeführt werden, vorzugsweise einstufig. Dabei wird bei der Absorption vorzugsweise als Absorber eine Einrichtung mit mehreren theoretischen Trennstufen verwendet, insbesondere 2 bis 8 theoretischen Trennstufen, besonders bevorzugt 3 bis 6.

Mögliche Ausführungsformen des Absorbers sind jeweils Kolonnen mit Böden, beispielsweise Glockenböden oder Siebböden, Kolonnen mit strukturierten Einbauten, wie beispielsweise Packungen, Kolonnen mit unstrukturierten Einbauten, wie beispielsweise Füllkörpern, oder Apparate in denen die Flüssigphase dispergiert vorliegt, beispielsweise durch Versprühen in Düsen, oder eine Kombination der genannten Ausführungsformen.

Die Desorption des Gasgemischs G-1 bzw. G-2 aus der Zusammensetzung Z-A bzw. Zusammensetzung Z-B gemäß Schritt (ii) oder (iv) des Verfahrens kann durch Druckabsenkung über dem Lösungsmittelgemisch, Temperaturerhöhung des Lösungsmittelgemischs oder durch Strippung mit Lösungsmitteldampf oder einer Kombination der genannten herbeigeführt werden.

Die Anforderungen an die Einrichtungen (Desorber) zur Desorption des Gasgemischs G-1 bzw. G-2 aus der Zusammensetzung Z-A bzw. Zusammensetzung Z-B, sowie die Führung der Phasen sind analog zu denen des Absorbers, d.h. geeignet sind Einrichtungen, in denen eine Gas-Flüssig-Phasengrenzfläche erzeugt wird, über die ein Stoff-und Wärmeübergang zwischen den Phasen ermöglicht wird, und die bei Bedarf mit internen oder externen Einrichtungen zur Wärmezufuhr und/oder Wärmeabfuhr versehen sind.

Die Desorption kann ein- oder mehrstufig durchgeführt werden. Mögliche Ausführungsformen des Desorbers sind ein einfacher (Entspannungs-)Behälter und Kolonnen.

Eine bevorzugte Ausführungsform, in der die Absorption, also das Inkontaktbringen mit dem Lösungsmittelgemisch, und die Desorption apparativ vereint sind, ist beispielsweise die Trennwandkolonne. Dabei wird das Inkontaktbringen, und damit verbunden die Absorption, und die Desorption durch Temperaturwechsel mehrstufig im Gegenstrom betrieben kombiniert mit einer Strippung mit Lösungsmitteldampf. Dabei kann sowohl gemäß (i) und (ii) als auch gemäß (iii) und (iv) eine apparative Vereinigung der Absorption und der Desorption erfolgen, insbesondere in einer Trennwandkolonne.

Im Sinne einer besonders bevorzugten Ausführungsform wird gemäß Schritt (i) zunächst das Gasgemisch G-0 enthaltend N₂O unter erhöhtem Druck p(Absorption) in einer im Gegenstrom betriebenen Absorptionskolonne mit Füllkörperschüttung mit dem Lösungsmittelgemisch LM-I in Kontakt gebracht, wobei eine Absorption stattfinden kann, und eine Zusammensetzung Z-A erhalten wird. Gemäß Schritt (ii) wird die Zusammensetzung Z-A gemäß dieser Ausführungsform in einen Behälter überführt, in dem die Zusammensetzung Z-A auf einen niedrigeren Druck p(Desoprtion) < p(Absorption) entspannt wird. Der Prozess wird vorzugsweise nahezu isotherm betrieben mit einer Temperaturdifferenz zwischen Absorptions- und Desorptionstemperatur von maximal 20 K, vorzugsweise maximal 15K, insbesondere maximal 10 K. Der Absorptionsdruck beträgt hierbei 1 bis 100 bar, bevorzugt 5 bis 65 bar, insbesondere 10 bis 40 bar, bevorzugt 10 bis 35 bar, besonders bevorzugt 13 bis 30 bar, weiter bevorzugt etwa 14 bis 25 bar und der Desorptionsdruck 0,1 bis 2 bar absolut, vorzugsweise 0,5 bis 1,5 bar absolut, besonders bevorzugt 1,0 bis 1,2 bar absolut.

Vorzugsweise wird ebenfalls gemäß Schritt (iii) zunächst das Gasgemisch G-1 unter erhöhtem Druck p(Absorption) in einer im Gegenstrom betriebenen Absorptionskolonne mit Füllkörperschüttung mit einem Lösungsmittelgemisch LM-II in Kontakt gebracht, wobei die Zusammensetzung Z-B erhalten wird. Zusammensetzung Z-B wird gemäß Schritt (iv) in einen Behälter überführt, in dem die Zusammensetzung Z-B auf einen niedrigeren Druck p(Desorption) < p(Absorption) entspannt wird. Der Prozess wird vorzugsweise ebenfalls nahezu isotherm betrieben mit einer Temperaturdifferenz zwischen Absorptions- und Desorptionstemperatur von maximal 20 K, vorzugsweise maximal 15 K, insbesondere maximal 10 K. Der Absorptionsdruck beträgt hierbei 1 bis 100 bar, bevorzugt 5 bis 65 bar, insbesondere 10 bis 40 bar, bevorzugt 10 bis 35 bar, besonders bevorzugt 13 bis 30 bar, weiter bevorzugt etwa 14 bis 25 bar und der Desorptionsdruck 0,1 bis 2 bar absolut, vorzugsweise 0,5 bis 1,5 bar absolut, besonders bevorzugt 1,0 bis 1,2 bar absolut.

Neben den Schritten (i), (ii), (iii) und (iv) kann Schritt (A) des Verfahrens auch weitere Schritte umfassen. So kann das Verfahren beispielsweise auch eine weitere Behandlung des Gasgemischs G-1 zwischen den Schritten (ii) und (iii) umfassen. Derartige Behandlungen umfassen beispielsweise eine Änderung der Temperatur oder eine Änderung des Drucks oder eine Änderung der Temperatur und des Drucks.

Dabei kann sich beispielsweise die Zusammensetzung eines Gasgemischs ändern, beispielsweise durch Kondensation einer der Komponenten. Bei diesen Komponenten kann es sich beispielsweise um Wasser oder eine andere im Lösungsmittelgemisch LM-I enthaltene Verbindung handeln, vorzugsweise um ein Lösungsmittel, das im Rahmen des Verfahrens im Lösungsmittelgemisch LM-I für Schritt (i) eingesetzt wird.

So ist es möglich, dass aus dem Gasgemisch G-1 oder G-2 weitere Komponenten abgetrennt werden. So ist es beispielsweise möglich, dass aus dem Gasgemisch G-2 durch Kompression und anschließendes Abkühlen Spuren von Wasser abgetrennt werden, die nach der Desorption gemäß Schritt (iv) im Gasgemisch G-2 enthalten sein können.

Dabei wird das Gasgemisch G-2 vorteilhafterweise auf einen Druck von 1 bis 35 bar komprimiert, bevorzugt 2 bis 30 bar, weiter bevorzugt 3 bis 27 bar. Eine Abkühlung erfolgt vorzugsweise anschließend, bevorzugt auf 1 bis 25 °C, besonders bevorzugt 3 bis 20 °C, insbesondere 4 bis 15°C, weiter bevorzugt 8 bis 12°C.

Dabei wird nach Schritt (A) im Rahmen von Schritt (B) eine Kondensation des gemäß Schritt (A) erhaltenen Gasgemischs G-A durchgeführt. Dabei wird eine flüssige Zusammensetzung Z-1 enthaltend Distickstoffmonoxid und ein gasförmiges Gemisch G-K erhalten, wobei das gasförmige Gemisch G-K vorzugsweise in die Behandlung gemäß Schritt (A) zurückgeführt wird.

Die Kondensation gemäß Schritt (B) des Verfahrens kann grundsätzlich nach jedem dem Fachmann bekannten geeigneten Verfahren erfolgen. Dabei ist es bevorzugt, dass das Gasgemisch G-A zumindest teilweise kondensiert wird. Dabei werden 20 bis 99 Gew.-%, bevorzugt 50 bis 90 Gew.-% und ganz besonders bevorzugt 60 bis 80 Gew.-% des Gasgemischs G-A kondensiert.

Dabei enthält das gasförmige Gemisch G-K beispielsweise 70 bis 90 Vol.-% Distickstoffmonoxid, insbesondere 75 bis 85 Vol.-%, besonders bevorzugt 78 bis 82 Vol.-%. Das gasförmige Gemisch G-K enthält darüber hinaus beispielsweise 4 bis 18 Vol.-% Kohlenstoffdioxid, insbesondere 6 bis 16 Vol.-%, besonders bevorzugt 8 bis 12 Vol.-% CO₂. Weiter enthält das gasförmige Gemisch G-K beispielsweise 0,01 bis 5 Vol.-% Sauerstoff, insbesondere 0,5 bis 3 Vol.-%, besonders bevorzugt 1,0 bis 2,0 Vol.-% Sauerstoff und beispielsweise 0 bis 1 Vol.-% Argon, wobei die Summe der Komponenten des gasförmigen Gemischs G-K 100 Vol.-% ergibt.

Vorzugsweise wird gemäß Schritt (B) das Gasgemisch G-A zunächst komprimiert und anschließend gekühlt. Dabei wird das Gasgemisch G-A vorteilhafterweise auf einen Druck von 1 bis 35 bar komprimiert, bevorzugt 2 bis 30 bar, weiter bevorzugt 3 bis 27 bar. Eine Abkühlung erfolgt vorzugsweise anschließend, bevorzugt auf 10 bis -70 °C, besonders bevorzugt 8 bis -30 °C, insbesondere 5 bis -25°C.

Sofern vor Schritt (B) des Verfahrens ein Schritt erfolgt, bei dem ein bereits komprimiertes Gasgemisch erhalten wird, umfasst Schritt (B) vorzugsweise keine weitere Kompression.

Das Verfahren zur Reinigung eines Gasgemischs enthaltend Distickstoffmonoxid kann auch weitere Schritte umfassen. So ist es auch möglich, dass das Verfahren weitere Schritte nach dem Schritt (B) umfasst.

Beispielsweise kann im Rahmen des Verfahrens die Zusammensetzung Z-1 weiter behandelt werden. Dabei ist es insbesondere möglich, dass ein weiterer Schritt zur

Aufkonzentrierung der Zusammensetzung Z-1 erfolgt. Dabei sind grundsätzlich alle dem Fachmann bekannten geeigneten Methoden zur weiteren Aufkonzentrierung der Zusammensetzung Z-1 oder zur Entfernung von Verunreinigungen wie beispielsweise von Resten von Lösungsmittel, möglich.

So umfasst das Verfahren insbesondere einen weiteren Schritt (C) zur Entfernung von Verunreinigungen aus der Zusammensetzung Z-1. Dabei wird vorzugsweise gemäß Schritt (C) die Zusammensetzung Z-1 enthaltend Distickstoffmonoxid mit einem Gasgemisch S-1 unter Erhalt einer Zusammensetzung Z-2 und eines Gasgemischs S-2 in Kontakt gebracht.

Als Gasgemisch S-1 können prinzipiell alle Substanzen eingesetzt werden die einen niedrigeren Siedepunkt als Distickstoffmonoxid haben oder Gemische davon. Bevorzugt werden Gase eingesetzt, die nicht mit Distickstoffmonoxid reagieren, beispielsweise Stickstoff, Helium, Neon, Argon, Krypton, Xenon, Wasserstoff, Kohlenstoffmonoxid, Methan und Tetrafluormethan. Bevorzugt wird Stickstoff eingesetzt.

Für die Behandlung gemäß Schritt (C) kann jeder Apparat verwendet werden, der dazu geeignet ist Gase und Flüssigkeiten miteinander in Kontakt zu bringen. Als Beispiele sind hier Blasensäulen, beispielsweise in Gleich- oder Gegenstrom betrieben, mit oder ohne Füllkörper bzw. Packung, in Riesel- oder Sumpf-Fahrweise, Rührkessel, beispielsweise mit Begasungsrührer, oder ähnliche zu nennen. Die Behandlung gemäß Schritt (C) kann sowohl in Batch oder kontinuierlich erfolgen. Bevorzugt wird sie kontinuierlich durchgeführt.

Die Behandlung gemäß Schritt (C) wird vorzugsweise bei einer Temperatur zwischen -90°C und +37°C, bevorzugt bei einer Temperatur zwischen -80°C und 0°C durchgeführt. Bevorzugt wird die Behandlung gemäß Schritt (C) bei einem Druck durchgeführt, der mindestens so hoch ist wie der Dampfdruck der flüssigen Zusammensetzung Z-1 bei der gewählten Temperatur und maximal bei 100 bar. Bevorzugt wird ein Druck gewählt, der 0,2 bis 5 bar oberhalb des Druckes bei dem die Absorption betrieben wird ist.

Die Menge an eingesetztem Gasgemisch S-1 muss groß genug sein, um die gewünschte Sauerstoff-Abreicherung zu erreichen, aber andererseits so klein wie möglich, um Verluste von Distickstoffmonoxid zu vermeiden. Typischerweise werden zwischen 5 und 100 mol Gasgemisch S-1 pro mol Sauerstoff in der flüssigen Zusammensetzung Z-1 eingesetzt, bevorzugt zwischen 15 und 30 mol Gasgemisch S-1 pro mol Sauerstoff in der flüssigen Zusammensetzung Z-1 eingesetzt.

Dabei wird gemäß Schritt (C) eine flüssige Zusammensetzung Z-2 erhalten, deren Gehalt an Sauerstoff gegenüber der flüssigen Zusammensetzung Z-1 weiter vermindert ist.

Dabei enthält die Zusammensetzung Z-2 beispielsweise 75 bis 95 Vol.-% Distickstoffmonoxid, insbesondere 80 bis 90 Vol.-%, besonders bevorzugt 82 bis 88 Vol.-%. Die Zusammensetzung Z-2 enthält darüber hinaus beispielsweise 4 bis 18 Vol.-% Kohlenstoffdioxid, insbesondere 6 bis 16 Vol.-%, besonders bevorzugt 8 bis 12 Vol.-% CO₂. Weiter enthält die Zusammensetzung Z-2 beispielsweise 0,01 bis 1,0 Vol.-% Sauerstoff, insbesondere 0,05 bis 0,5 Vol.-%, besonders bevorzugt 0,1 bis 0,4 Vol.-% Sauerstoff und beispielsweise 0 bis 1 Vol.-% Stickstoff, wobei die Summe der Komponenten der Zusammensetzung Z-2 100 Vol.-% ergibt.

In Schritt (C) wird weiter ein Gasgemisch S-2 erhalten, das neben dem Gasgemisch S-1 weitere Komponenten enthalten kann, beispielsweise Sauerstoff.

Dabei enthält das Gasgemisch S-2 beispielsweise 70 bis 90 Vol.-% Distickstoffmonoxid, insbesondere 75 bis 85 Vol.-%, besonders bevorzugt 77 bis 82 Vol.-%. Das Gasgemisch S-2 enthält darüber hinaus beispielsweise 4 bis 18 Vol.-% Kohlenstoffdioxid, insbesondere 6 bis 16 Vol.-%, besonders bevorzugt 8 bis 12 Vol.-% CO₂. Das Gasgemisch enthält beispielsweise 4 bis 18 Vol.-% Stickstoff, insbesondere 6 bis 16 Vol.-%, besonders bevorzugt 8 bis 12 Vol.-% Stickstoff. Weiter enthält das Gasgemisch S-2 beispielsweise 0,01 bis 5 Vol.-% Sauerstoff, insbesondere 0,5 bis 3 Vol.-%, besonders bevorzugt 1,0 bis 2,0 Vol.-% Sauerstoff und beispielsweise 0 bis 1 Vol.-% Argon, wobei die Summe der Komponenten des Gasgemischs S-2 100 Vol.-% ergibt.

Dabei ist es möglich, dass das Gasgemisch S-2 in eine Stufe des Verfahrens zurückgeführt wird. Gemäß einer derartigen Ausführungsform kann Distickstoffmonoxid, das in dem Gasgemisch S-2 enthalten sind, in das Verfahren zurückgeführt werden, um Ausbeuteverluste zu vermeiden.

Dabei wird das Gasgemisch S-2 vorzugsweise in den Schritt (A) des Verfahrens zurückgeführt. Dabei wird das Gasgemisch S-2 mit einem anderen Gasgemisch gemischt. Vorzugsweise wird dabei das Gasgemisch S-2 derart in den Schritt (A) zurückgeführt, dass eine Gewinnung des gegebenenfalls im Gasgemisch S-2 enthaltenen Distickstoffmonoxids möglich ist. Daher ist es bevorzugt, dass das Gasgemisch S-2 mit einem Gasgemisch gemischt wird, das einer Absorption zugeführt wird, insbesondere dem Gasgemisch G-0 oder dem Gasgemisch G-1. Somit ist es bevorzugt, das Gasgemisch S-2 in Schritt (i) oder in Schritt (iii) des Schritts (A) zurückzuführen.

Die Oxidation von Cyclopenten mittels Distickstoffmonoxid bzw. mittels eines Gasgemischs enthaltend Distickstoffmonoxid kann generell gemäß sämtlichen Verfahrensführungen erfolgen, bei denen die Oxidation stattfindet. Insbesondere sind sowohl kontinuierliche Verfahrensführungen und Fahrweisen der Umsetzung als auch Batch-Reaktion möglich. Die Reaktionsbedingungen für die Oxidation von Cyclopenten werden erfindungsgemäß so gewählt, dass eine Reaktion stattfindet. Druck und Temperatur können entsprechend gewählt werden.

Vorzugsweise liegt der Druck in einem Bereich von bis zu 500 bar, beispielsweise 1 bis 320 bar, bevorzugt 10 bis 300 bar, insbesondere 90 bis 280 bar. Die Temperatur liegt vorzugsweise in einem Bereich von 180 bis 320°C, beispielsweise 200 bis 300°C, insbesondere 240 bis 290°C.

Dabei kann die Oxidation von Cyclopenten in Gegenwart eines geeigneten Lösungsmittels durchgeführt werden. Es ist aber ebenso möglich, die Oxidation ohne den Zusatz eines Lösungsmittels durchzuführen.

Vorzugsweise wird die Oxidation von Cyclopenten durch geeignete Wahl des Drucks und der Temperatur so geführt, dass in der Reaktionszone keine Gasphase auftritt.

Die Umsetzung von Cyclopenten und Distickstoffmonoxid wird dabei vorzugsweise adiabatisch durchgeführt.

Dabei wird unter einer adiabatisch geführten Umsetzung eine Umsetzung verstanden, bei der während der Reaktion im Wesentlichen kein Wärmeaustausch zwischen Reaktorinhalt und Umgebung stattfindet. Bevorzugt wird im Rahmen der vorliegenden Erfindung unter einer adiabatisch geführten Umsetzung eine Umsetzung verstanden, bei der bevorzugt weniger als 10 %, besonders bevorzugt weniger als 5% der erzeugten Wärme an die Umgebung abgegeben wird.

Dabei wird das Verfahren vorzugsweise so durchgeführt, dass Cyclopenten und Distickstoffmonoxid in einem thermisch gegenüber der Umgebung isolierten Reaktor umgesetzt werden, wobei die bei der exothermen Reaktion erzeugte Wärmeenergie im Wesentlichen im Reaktor verbleibt und nicht nach außen abgeführt wird. Ein geeignetes Verfahren ist beispielsweise in WO 2010/023211 beschrieben.

Bei einer adiabatischen Verfahrensführung ist die Differenz zwischen der Temperatur der Produkte (T(aus)) und der Temperatur der Edukte (T(ein)) als adiabatische Temperaturerhöhung (T(adiab)) definiert. In einer bevorzugten Ausführungsform des Verfahrens beträgt T(adiab) zwischen 10 und 140 °C, besonders bevorzugt zwischen 20 und 125 °C und ganz besonders bevorzugt zwischen 25 und 100 °C.

Das Verfahren kann beispielsweise derart durchgeführt werden, dass geeignete Parameter, wie beispielsweise die Umsätze der einzelnen Edukte, die wiederum durch die Verweilzeit, durch die Eingangstemperatur des Eduktgemisches (T(ein)), durch den Reaktionsdruck und durch die Konzentrationen der einzelnen Edukte im Eduktgemisch beeinflusst werden, so eingestellt werden, dass die durch die Reaktion erzeugte Reaktionswärme die Wärme ist, welche notwendig ist, damit das Produktionsgemisch den Reaktor mit einer Temperatur (T(aus)) verlässt, die mindestens 10 K unterhalb der Temperatur liegt, bei der die adiabatische Induktionszeit genau 24 Stunden beträgt. Die adiabatische Induktionszeit in Abhängigkeit der Temperatur kann auf an sich bekannte Weise aus den Daten von DSC-Experimenten mit unterschiedlichen Aufheizraten abgeleitet werden.

Dabei ist es möglich, dass beide Edukte die gleiche oder verschiedene Eingangstemperatur(en) aufweisen. Relevant ist im Rahmen der vorliegenden Erfindung die Reaktoreingangstemperatur des Eduktgemischs, also die Temperatur, die sich einstellt, wenn alle Eduktströme zusammengemischt werden.

In einer bevorzugten Ausführungsform beträgt die Reaktoreingangstemperatur des Eduktgemischs (T(ein)) 170 bis 270 °C, besonders bevorzugt 200 bis 260 °C, beispielsweise 220 bis 250 °C.

Die Temperatur, die die Edukte am Reaktoreingang aufweisen, entspricht bevorzugt auch der minimalen Temperatur, bei der im erfindungsgemäßen Verfahren der gewünschte Umsatz noch in einer technisch realisierbaren Reaktorgröße erzielt werden kann. Somit beträgt die minimale Temperatur, bei der im erfindungsgemäßen Verfahren der gewünschte Umsatz noch in einer technisch realisierbaren Reaktorgröße erzielt werden kann, im Allgemeinen wenigstens 170 °C, bevorzugt wenigstens 200 °C.

Die maximale Reaktorausgangstemperatur (T(aus)) des Produktgemischs, bei der das Verfahren durchgeführt werden kann, beträgt im Allgemeinen höchstens 340 °C, bevorzugt höchstens 320 °C, besonders bevorzugt höchstens 300 °C. Die maximale Reaktorausgangstemperatur (T(aus)) wird dabei so gewählt, dass bevorzugt keine thermische Zersetzung des gebildeten Produktes bzw. der nicht umgesetzten Edukte stattfindet.

Somit wird das Verfahren im Allgemeinen bei einer Temperatur von 170 bis 340 °C, bevorzugt 200 bis 320 °C, durchgeführt, wobei die erstgenannte Temperatur die Reaktoreingangstemperatur (T(ein)) des Eduktgemischs und die zweitgenannte Temperatur die Reaktorausgangstemperatur (T(aus)) des Produktgemischs ist.

In einer bevorzugten Ausführungsform wird das Verfahren bei einem Reaktionsdruck von 60 bis 500 bar, besonders bevorzugt von 80 bis 325 bar, besonders bevorzugt von 90 bis 180 bar, beispielsweise bei 100 bis 150 bar, durchgeführt.

Das Verfahren kann derart durchgeführt werden, dass das molare Verhältnis zwischen Cyclopenten und Distickstoffmonoxid einen geeigneten Wert hat, so dass die durch die Reaktion erzeugte Reaktionswärme genau die Wärme ist, welche bei einer entsprechenden Reaktoreingangstemperatur (T(ein)) des Eduktgemischs und bei Vollumsatz des im Unterschuss vorliegenden Eduktes, bevorzugt Distickstoffmonoxid, eine Reaktorausgangstemperatur (T(aus)) des Produktgemischs ergibt, die unterhalb der oben genannten maximalen Temperaturen von 340 °C, bevorzugt 320 °C, besonders bevorzugt 300 °C, liegt.

In einer bevorzugten Ausführungsform beträgt das molare Verhältnis von Distickstoffmonoxid zu Cyclopenten im Bereich von 0,02:1 bis 0,3:1, besonders bevorzugt von 0,05:1 bis 0,25:1, und ganz besonders bevorzugt von 0,08:1 bis 0,2:1. Dabei wird unter dem "molaren Verhältnis der Edukte" der Quotient der Stoffmengen der Edukte verstanden.

In einer weiteren bevorzugten Ausführungsform liegt in dem Verfahren der Umsatz bezüglich Distickstoffmonoxid im Bereich von 80 bis 100%, besonders bevorzugt von 90 bis 99%, ganz besonders bevorzugt von 90 bis 96%.

Das Verfahren kann in allen dem Fachmann bekannten Reaktoren durchgeführt werden, die für eine adiabatische Reaktionsführung geeignet sind, beispielsweise in einem Rohrreaktor. Um eine adiabatische Reaktionsführung zu gewährleisten, ist es beispielsweise notwendig, dass der Reaktor ausreichend gegenüber der Umgebung isoliert ist, so dass im Wesentlichen keine Reaktionswärme an die Umgebung abgegeben wird und somit der eigentlichen Umsetzung nicht mehr zur Verfügung steht. In einer besonders bevorzugten Ausführungsform wird die durch die Reaktion erzeugte Wärme durch den Produktstrom aus dem Reaktor ausgetragen.

Dabei ist es auch möglich, mehrere Reaktoren zu verwenden, die parallel oder in Serie geschaltet sein können.

Der Reaktorraum des einsetzbaren Reaktors kann leer sein oder gegebenenfalls durch geeignete Einbauten segmentiert sein. Im Allgemeinen weist der Reaktor ein für eine adiabatisch geführte Reaktion geeignetes Strömungsprofil auf. In dem einzusetzenden Reaktor findet bevorzugt im Wesentlichen keine Rückvermischung statt. Bevorzugt hat der Reaktor eine Verweilzeitverteilung die der einer Rührkesselkaskade mit mindestens 8 Rührkesseln entspricht. Besonders bevorzugt hat der Reaktor eine Verweilzeitverteilung die der einer Rührkesselkaskade mit mindestens 12 Rührkesseln entspricht. Das für das Verfahren bevorzugte Strömungsprofil der Reaktionsmischung ist abhängig vom eingesetzten Reaktor und kann gegebenenfalls durch geeignete, dem Fachmann bekannte Einbauten, beispielsweise Lochbleche, oder durch Füllung des Reaktors mit einer geeigneten Schüttung, entsprechend eingestellt werden.

Bevorzugt wird ein Rohrreaktor eingesetzt, mit einem Längen zu DurchmesserVerhältnis größer 1 eingesetzt. Besonders bevorzugt enthält der Reaktor mindestens Lochbleche zur Verringerung der Rückvermischung.

Es ist möglich, dass der Reaktor liegend oder stehend betrieben wird, bevorzugt stehend. Ein stehender Reaktor kann von unten nach oben oder von oben nach unten von dem Reaktionsgemisch durchströmt werden. Bevorzugt wird das Verfahren in einem stehenden Reaktor, der von unten nach oben von dem Reaktionsgemisch durchströmt wird, durchgeführt.

Ein für die kontinuierliche Verfahrensführung besonders geeigneter Reaktor ist beispielsweise ein Rohrreaktor, der bevorzugt ausreichend isoliert ist. Entsprechende Rohrreaktoren sind dem Fachmann bekannt.

Dabei ist es möglich, dass die Eduktströme dem Reaktor separat zugeführt werden. Es ist erfindungsgemäß auch möglich und bevorzugt, dass die Eduktströme bereits vorgemischt dem Reaktor zugeführt werden.

Die Eduktströme oder zumindest ein Teil der eingesetzten Eduktströme, beispielsweise 70 bis 95%, können nach allen dem Fachmann bekannten Verfahren vor der Umsetzung auf eine Temperatur von bevorzugt 170 bis 270 °C, besonders bevorzugt 200 bis 260 °C, beispielsweise 220 bis 250 °C, vorgewärmt werden, beispielsweise durch eine externe Wärmequelle, beispielsweise Wasserdampf, in einem dem Fachmann bekannten Wärmetauscher, der als Vorwärmer fungiert. Das Vorwärmen der Eduktströme erfolgt vorzugsweise außerhalb des Reaktors in einem geeigneten Wärmeaustauscher.

Dabei wird die zum Vorwärmen der Eduktströme notwendige Wärmeenergie vorzugsweise zumindest teilweise, bevorzugt vollständig, aus dem Reaktoraustrag, d. h. aus dem heißen Produktstrom des Verfahrens, entnommen. Dazu wird in einem Wärmetauscher, beispielsweise einem Gegenstrom-Wärmetauscher, wenigstens ein Teil des Produktstromes mit wenigstens einem Teil, beispielsweise 70 bis 95%, des Eduktgemischs in Kontakt gebracht.

Die Temperatur des dem Reaktor zugeführten Stroms kann beispielsweise über den Anteil Eduktstroms, der über einen derartigen Wärmeaustauscher vorgewärmt wird, eingestellt werden.

Dabei weist der aus dem Verfahren erhaltene Produktstrom eine Reaktorausgangstemperatur (T(aus)) von im Allgemeinen höchstens 340 °C, bevorzugt höchstens 320 °C, besonders bevorzugt höchstens 300 °C auf. Nach Inkontaktbringen mit dem Eduktstrom weist der Produktstrom im Allgemeinen eine Temperatur von 150 bis 220 °C, bevorzugt 170 bis 200 °C, beispielsweise 180 bis 190 °C, auf. Der Eduktstrom wird auf im Allgemeinen 180 bis 280 °C, bevorzugt 240 bis 275 °C, beispielsweise 250 bis 260 °C, erwärmt.

Gemäß diesem Verfahren wird als Produktstrom bevorzugt ein Reaktionsgemisch erhalten, welches wenigstens Cyclopentanon und Stickstoff, enthält. Neben diesen gewünschten Produkten liegen in dem Gemisch beispielsweise nicht umgesetzte Edukte und/oder Nebenprodukte vor.

Das erhaltene Cyclopentanon, bzw. das derart erhaltene Reaktionsgemisch enthaltend Cyclopentanon, kann prinzipiell in der erhaltenen Form weiter verarbeitet werden. Ebenso kann das erhaltene Gemisch jedoch auch gemäß sämtlichen geeigneten Verfahren zur Gewinnung des Cyclopentanon, aufgearbeitet werden. Besonders bevorzugt sind destillative Verfahren zur Aufarbeitung.

Nach der Reaktion von Cyclopenten mit N₂O wird der Reaktorinhalt abgekühlt und entspannt, wobei das Abkühlen und Entspannen in beliebiger Reihenfolge und ein- oder mehrstufig erfolgen kann. Dabei ist es im Rahmen der vorliegenden Erfindung auch möglich, dass schrittweise abgekühlt und entspannt wird. Dabei wir das gebildete Stickstoff und das nicht umgesetzte N₂O bereits zum größten Teil als Abgas abgetrennt. Die flüssigen organischen Komponenten werden dann einer Destillation unterworfen, um unumgesetztes Cyclopenten zurückzugewinnen.

Dabei kann die Aufarbeitung einen oder mehrere Reinigungsschritte umfassen, beispielsweise mindestens eine Destillation, bevorzugt aber mindestens eine einstufige Verdampfung, beispielsweise zur Abtrennung von N₂ und nicht umgesetztem Distickstoffmonoxid, und mindestens eine Destillation, besonders bevorzugt mindestens eine einstufige Verdampfung und mindestens zwei Destillationsschritte.

In der Aufarbeitung wird das Produkt Cyclopentanon von nicht umgesetztem Cyclopenten und gegebenenfalls in der Reaktionsmischung vorliegendem Cyclopentan getrennt.

Dabei wird das erhaltene Gemisch zunächst in wenigstens einem geeigneten Behälter auf einen Druck entspannt, der im Allgemeinen unterhalb des Reaktionsdruckes liegt, beispielsweise auf einen Druck von 1 bis 20 bar, bevorzugt 14 bis 18 bar. In einer bevorzugten Ausführungsform wird das Gemisch vor diesem Entspannen in einem geeigneten Wärmetauscher abgekühlt.

Weiter umfasst die Aufarbeitung vorzugsweise mindestens einen Destillationsschritt in einer geeigneten Destillationskolonne, vorzugsweise in einer Rückführkolonne.

Geeignet ist beispielsweise eine Rückführkolonne, die 30 bis 50, bevorzugt 35 bis 45, theoretische Böden aufweist. Der Zulauf erfolgt im Allgemeinen im mittleren Teil der Kolonne. In einer weiteren bevorzugten Ausführungsform wird beispielsweise Cyclopenten in einem Seitenabzug der Kolonne gewonnen.

Da sich bei Oxidation von Cyclopenten mit N₂O auch Leichtsieder bilden, wie z.B. Aceton und Ethylen (aus der Oxidation von 2-Methyl-2-buten, das in Cyclopenten als Verunreinigung enthalten ist), wird zweckmäßiger Weise das Rück-Cyclopenten nicht als Kopfstrom sondern als Seitenstrom entnommen. Als Kopfstrom wird nur ein kleiner Purge-Strom entnommen, der als Ausschleusung für Leichtsieder wie beispielsweise Aceton dient. Die Destillation wird bei leicht erhöhtem Druck, beispielsweise bei 2 bis 6 bar, insbesondere bei 3 bis 5 bar, bevorzugt bei etwa 4 bar, durchgeführt. Die Kolonne hat insgesamt beispielsweise 30 bis 50, bevorzugt beispielsweise 35, 36, 37, 38, 39, 40, 41, 42, 43 oder 44 Trennstufen, besonders bevorzugt 38, 39 oder 40 Trennstufen. Unterhalb des Zuströmpunkts des Feeds liegen beispielsweise 17 bis 22 Trennstufen, bevorzugt 18, 19 oder 20 Trennstufen. Zwischen Zuströmpunkt des Feeds und Seitenabzug liegen beispielsweise 7 bis 12 Trennstufen, bevorzugt 8, 9 oder 10 Trennstufen und zwischen Seitenabzug und Kopf liegen beispielsweise 8 bis 14 Trennstufen, bevorzugt 10, 11 oder 12 Trennstufen. Die Kolonne wird so eingestellt, dass sich das Cyclopentan im Kreislauf aufpegelt. Im stationären Zustand enthält das Rück-Cyclopenten, das bevorzugt im Seitenabzug abgenommen wird, zwischen 40 und 60 Gew.-% Cyclopentan. Im Sumpf der Kolonne wird "crude" Cyclopentanon mit einer Reinheit von >80 Gew.-%, bevorzugt >90 Gew.-% abgetrennt. In diesem Strom sind außerdem noch Cyclopentan und andere Nebenprodukten aus der Oxidation enthalten. Ein geeigneter Strom enthaltend abdestilliertes nicht umgesetztes Cyclopenten, kann dann beispielsweise zurückgeführt werden und alleine oder nach Zusatz eines geeigneten Gemischs enthaltend Cyclopenten, wieder im Verfahren eingesetzt werden.

Die Destillation in der Rückführkolonne erfolgt beispielsweise bei einem Druck von 1,0 bis 7,0 bar, bevorzugt von 2,0 bis 6,0 bar, beispielsweise von 3,5 bis 5,0 bar.

Die Destillation in der Rückführkolonne erfolgt beispielsweise bei einer Temperatur von 80 bis 200 °C, bevorzugt von 90 bis 190 °C. Dabei liegt die Temperatur im Sumpf der Kolonne beispielsweise im Bereich von 150 bis 200 °C, bevorzugt von 160 bis 185 °C, die Temperatur oberhalb des Sumpfes der Kolonne liegt beispielsweise im Bereich von 80 bis 110 °C, bevorzugt von 90 bis 105 °C.

Gemäß einer weiteren Ausführungsform der Aufarbeitung wird nicht umgesetztes Cyclopenten in Mischung mit weiteren Kohlenwasserstoffen, beispielsweise Cyclopentan, erhalten, beispielsweise als eine Mischung enthaltend 20 bis 98 Gew.-%, bevorzugt 30 bis 80 Gew.-%, besonders bevorzugt 40 bis 60 Gew.-%, jeweils bezogen auf die Mischung, Cyclopenten, und 2 bis 80 Gew.-%, bevorzugt 20 bis 70 Gew.-%, besonders bevorzugt 40 bis 60 Gew.-%, jeweils bezogen auf die Mischung, wenigstens einen weiteren Kohlenwasserstoff, beispielsweise einen gesättigten Kohlenwasserstoff, insbesondere Cyclopentan. Diese Mischung kann weitere Komponenten, beispielsweise Kohlenwasserstoffe, Produkt bzw. Nebenprodukt aus der Umsetzung und/oder lineare Olefine, bis zu einem Gesamtgehalt von bis zu 1,5 Gew.-%, bevorzugt bis zu 1,0 Gew.-%, jeweils bezogen auf die Mischung, enthalten.

In einer weiteren bevorzugten Ausführungsform der Aufarbeitung werden am Kopf der Rückführkolonne niedrig siedende Komponenten erhalten, beispielsweise C5-Kohlenwasserstoffe wie n-Pentan, 2-Methyl-2-buten, cis-2-Penten, und trans-2-Penten.

Cyclopentanon wird gemäß einer weiteren Ausführungsform der Aufarbeitung im Sumpf der Rückführkolonne erhalten, in einer bevorzugten Ausführungsform mit einer Reinheit von bis zu 95 Gew.-%, bevorzugt bis zu 92 Gew.-%, jeweils bezogen auf die Sumpffraktion.

Dabei ist es auch möglich, dass die Aufarbeitung neben der einstufigen Verdampfung und der ersten Destillation, vorzugsweise der Destillation in einer Rückführkolonne, eine weitere Destillation umfasst. So ist es möglich, dass zur weiteren Aufreinigung des Produktes das Cyclopentanon, destilliert werden kann, beispielsweise in einer oder mehreren Kolonnen, bevorzugt in zwei Kolonnen oder besonders bevorzugt in einer Trennwandkolonne.

Die Aufreinigung des aus der Destillation in der Rückführkolonne erhaltenen Produktes erfolgt beispielsweise bei einem Druck von 0,5 bis 3 bar, bevorzugt 0,8 bis 2 bar, beispielsweise 1,0 bis 1,2 bar.

Die Aufreinigung des aus der Destillation in der Rückführkolonne erhaltenen Produktes erfolgt beispielsweise bei einer Temperatur von 100 bis 200 °C, bevorzugt 110 bis 180 °C, beispielsweise 120 bis 170 °C.

Beispielsweise erfolgt die Aufreinigung des aus der Destillation in der Rückführkolonne erhaltenen Produktes beispielsweise in einer Trennwandkolonne bei einem Druck von 0,5 bis 3 bar, bevorzugt 0,8 bis 2 bar, beispielsweise 1,0 bis 1,2 bar und bei einer Temperatur von 100 bis 200 °C, bevorzugt 110 bis 180 °C, beispielsweise 120 bis 170 °C

Das Sumpfprodukt aus der ersten Kolonne wird dann destillativ weiter aufgearbeitet, um daraus reines Cyclopentanon zu gewinnen. Für diese destillative Reinigung von Cyclopentanon können zwei Kolonnen eingesetzt werden. In der ersten Kolonne mit beispielsweise 17 bis 25 Trennstufen, bevorzugt 19 bis 23 Trennstufen, insbesondere 20, 21 oder 22 Trennstufen werden bei 0,9 bis 1,3 bar, bevorzugt 1,0 bis 1,2 bar, besonders bevorzugt bei 1,1, bar die leichtsiedenden Nebenkomponenten über Kopf abgetrennt. Das Sumpfprodukt wird dann in einer weiteren Kolonne mit beispielsweise 30 bis 50 Trennstufen, bevorzugt 35 bis 44 Trennstufen, insbesondere 36, 37, 38, 39 oder 40 Trennstufen bei 0,9 bis 1,3 bar, bevorzugt 1,0 bis 1,2 bar, besonders bevorzugt bei 1,1, bar destilliert, wobei reines Cyclopentanon über Kopf gewonnen wird und schwersiedenden Verunreinigungen über Sumpf abgetrennt werden. Besonders bevorzugt ist es allerdings, wenn die destillative Reinigung von Cyclopentanon in einer einzigen Trennwandkolonne durchgeführt wird

Das reine Cyclopentanon, das aus dem Seitenabzug gewonnen wird, hat eine Reinheit von mindestens 99 Gew.-%, bevorzugt mindestens 99,5 Gew.-% und besonders bevorzugt mindestens 99,8 Gew.-%.

Als Kopfprodukt wird ein Strom erhalten, der alle leichtsiedenden Nebenkomponenten enthält, aber maximal 0,1 Gew.-% Cyclopentanon, bevorzugt maximal 0,01 Gew.-% Cyclopentanon. Als Hauptkomponenten sind darin enthalten Cyclopentan, 4-Pentenal, 3-Methyl-2-butanon (aus der Oxidation von 2-Methyl-2-buten), Cyclopentenoxid und Cyclopenten (jeweils >5 Gew.-%). Als Nebenkomponenten sind außerdem noch Aceton, 2-Methyl-2-buten, 2-Methyl-1-buten, 3-Methylpentan, Pivalaldehyd (aus der Oxidation von 2-Methyl-2-buten), Methylcyclopentan, Diethylketon (aus der Oxidation von 2-Penten), Cyclopropylacetaldehyd, Cyclobutylcarbaldehyd, 2-Methyl-3-pentanon (aus der Oxidation von 2-Methyl-2/3-hexen) und Cyclopentenon in diesem Strom enthalten.

Als Sumpfprodukt erhält man einen Strom mit schwersiedenden Verunreinigungen. Dieser Strom enthält noch maximal 50 Gew.-% Cyclopentanon, bevorzugt maximal 40 Gew.-% Cyclopentanon. Als Hauptkomponente enthält dieser Strom Cyclopenten-Dimere (Cyclopentylcyclopentene) und Cyclopentanon-Dimere.

Der Kopfstrom dieser Kolonne, der zwischen 10 und 50 Gew.-% 4-Pentenal enthält, kann entweder direkt oder nach weiteren Behandlungen als Gemisch (G) in das erfindungsgemäße Verfahren zur Herstellung von 4-Pentensäure eingesetzt werden.

Bevorzugt wird dieser Strom vor der Oxidation durch Destillation weiter aufkonzentriert. Ganz besonders bevorzugt werden vor der Oxidation die darin enthaltenen Leichtsieder Cyclopenten und Cyclopentan weitgehend entfernt.

Das derart erhaltene Gemisch (G) enthaltend 4-Pentenal, 3-Methyl-2-butanon und Cyclopentenoxid eignet sich insbesondere zur Herstellung von 4-Pentensäure.

Gemäß einem weiteren Aspekt betrifft die vorliegende Erfindung daher auch die Verwendung eines Gemischs (G) enthaltend 4-Pentenal, 3-Methyl-2-butanon und Cyclopentenoxid zur Herstellung von 4-Pentensäure.

Insbesondere geeignet ist wie ausgeführt ein Gemisch (G), enthaltend 4-Pentenal, 3-Methyl-2-butanon und Cyclopentenoxid, das als Nebenprodukt der Oxidation von Cyclopenten zu Cyclopentanon mittels Distickstoffmonoxid erhalten wird.

Daher betrifft die vorliegende Erfindung gemäß einer weiteren Ausführungsform auch die Verwendung eines Gemischs (G) enthaltend 4-Pentenal, 3-Methyl-2-butanon und Cyclopentenoxid zur Herstellung von 4-Pentensäure wie oben beschrieben, wobei das Gemisch (G) als Nebenprodukt der Oxidation von Cyclopenten zu Cyclopentanon mittels Distickstoffmonoxid erhalten wird.

### Figurenbeschreibung

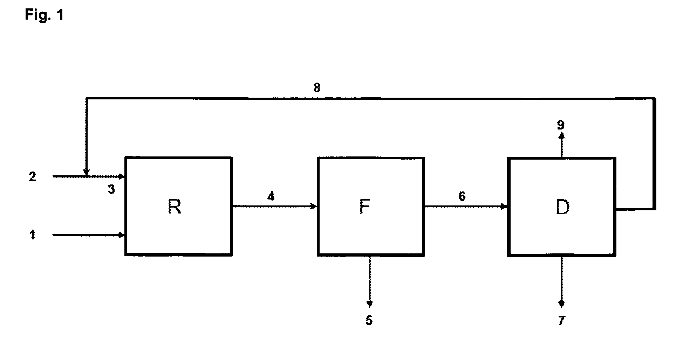

- Fig.1: zeigt den schematischen Aufbau einer Anlage zur Umsetzung von Cyclopenten mit Distickstoffmonoxid umfassend einen Reaktor **(R),** einen Flash-Behälter **(F)** und eine Destillationskolonne **(D).** Dabei wird über Strom (1) N₂O zum Reaktor dosiert, über Strom **(2)** wird der frische Cyclopenten-Feed zudosiert. Strom **(2)** wird mit Strom **(8)** gemischt (Rück-Cyclopenten), um einen Strom **(3)** zu erzeugen. Strom **(4)** entspricht dem Reaktoraustrag, der dem Flash-Behälter zugeführt wird. Aus dem Flash-Behälter werden ein gasförmiger Strom **(5)** und eine flüssige Phase **(6)** entnommen. Strom **(6)** wird der Destillationskolonne **(D)** zugeführt. Aus der Destillationskolonne werden als Sumpfprodukt Strom **(7),** als Seitenabzugsprodukt Strom **(8)** und als Kopfstrom Strom **(9)** erhalten.

Im Folgenden wird die Erfindung anhand von Beispielen näher erläutert.

### Beispiele

### Beispiel 1: Umsetzung von Cyclopenten mit Distickstoffmonoxid

Der Versuch gemäß Beispiel 1 wurde in einer Anlage mit einem Aufbau gemäß der schematischen Figur 1 durchgeführt.

Durch Strom **(2)** wurde der frische Cyclopenten-Feed mit 116,4 g/h zudosiert. Dieser stammte aus der Destillation eines C₅-Schnitts eines Steamcrackers und hatte folgende Zusammensetzung (Gew.-%): Cyclopenten (ca. 95,1 %), Cyclopentan (ca. 3,4 %), 2-Methyl-2-buten (ca. 1,2 %).

Dieser Strom wurde zunächst mit Strom **(8)** gemischt (Rück-Cyclopenten), um einen Strom **(3)** zu erzeugen, der folgende Zusammensetzung hatte: Cyclopenten (ca. 46,3 %), Cyclopentan (ca. 51,9 %), 2-Methyl-2-buten (ca. 0,9 %), 2,2-Dimethylbutan (ca. 0,81 %).

Dieser Strom wurde dann mit einer Dosierpumpe zum Reaktor **(R)** dosiert (Mengenstrom: ca. 2076 g/h). Über Strom (1) wurde flüssiges N₂O (Gehalt an N₂O > 99,8 Vol.-%, Firma Messer Griesheim) mit ca. 74 g/h zum Reaktor dosiert. Das molare Verhältnis Cyclopenten:N₂O im Reaktorfeed betrug 0,11 mol/mol. Der Reaktor bestand aus einem Rohr (Außendurchmesser = 60,3 mm, Wanddicke = 2,4 mm, Länge = ca. 4 m). Das Reaktionsvolumen betrug (abzüglich des Volumens von Füllkörpern) inklusive Verbindungsstücke insgesamt ca. 8 L.

Das Rohr war mit einem Isoliermantel mit zusätzlicher dreigeteilter Stützheizung versehen, die auf (von unten) 256 °C, 275 °C und 317 °C eingestellt war. Der Cyclopenten-Umsatz im geraden Durchgang betrug 11 % und der N₂O-Umsatz ca. 96 %. Der Reaktoraustrag **(4)** wurde nach der Druckhaltung in zwei Schritten mit zwei bei 10 bar und 1 bar betriebenen Flash-Behältern **(F)** auf 1 bar entspannt und abgekühlt. Die gasförmigen Komponenten (Strom **(5))** wurden abgetrennt, und in einem Nachkühler (bei +5°C betrieben, nicht im Schema gezeigt) wurden darin enthaltenen Kohlenwasserstoffe möglichst vollständig auskondensiert.

Die flüssige Phase **(6)** wurde in einer Destillationskolonne **(D)** (Glockenbodenkolonne mit 20 Böden und flüssigem Seitenabzug) aufgetrennt. Als Sumpfprodukt (7) erhielt man 138,7 g/h eines Stroms mit folgender Zusammensetzung: Cyclopentanon (ca. 95,3 Gew.-%), Cyclopentan (ca. 0,8 Gew.-%), 4-Pentenal (ca. 1,3 Gew.-%), Cyclopentenoxid (ca. 0,37 Gew.-%), Cyclopenten-Dimere (ca. 0,53 Gew.-%), Cyclopenten (ca. 0,08 Gew.-%).

Das Seitenabzugsprodukt, Strom **(8),** das 45,6 % Cyclopenten enthielt, wurde über Strom (3) zum Reaktor zurückgeführt.

Am Kopf der Kolonne wurden über den Kopfstrom **(9)** lediglich geringste Mengen Leichtsieder (z.B. Ethylen und Acetaldehyd aus der Oxidation von 2-Methyl-2-buten) ausgeschleust.

### Beispiel 2: Destillative Reinigung von Cyclopentanon unter Erhalt eines 4-Pentenal-reichen Stroms

Aus der in Beispiel 1 beschriebenen Anlage wurde aus einem längeren Lauf das Produkt gesammelt. Ingesamt wurden für die Destillation 35 kg gesammelt, die folgende Zusammensetzung aufwiesen: Cyclopentanon (95,5 Gew.-%), Cyclopentan (1,0 Gew.-%), 4-Pentenal (1,3 Gew.-%), Cyclopentenoxid (0,4 Gew.-%), 3-Methyl-2-butanon (0,3 Gew.-%), Cyclopenten-Dimere (0,5 Gew.-%), 2-Cyclopentylcyclopentanon (0,5 Gew.-%), 3-Methylpentan (0,1 Gew.-%) und Cyclopenten (0,1 Gew.-%) neben einer Reihe von weiteren Nebenprodukten mit Konzentrationen jeweils unter 100 ppm.

Dieses Gemisch wurde in einer kontinuierlich betriebenen Labortrennwandkolonne aufdestilliert. Die Kolonne hatte einen Durchmesser von 43 mm und eine Packungshöhe von 2,5 m und war mit einer Packung versehen (Montz A3 1000). Zwischen 0,85 und 2,10 m oberhalb der Unterkante der Packung war die Kolonne durch ein mittig platziertes Trennblech getrennt. Der Produktzulauf befand sich 1,0 m oberhalb der Packungsunterkante. Der Seitenabzug befand sich auf 1,3 m oberhalb der Packungsunterkante, aber auf der anderen Seite des Trennbleches. Die Destillation wurde bei einem Kopfdruck von 0,6 bar durchgeführt. Der Feed (330 g/h) wurde vorgeheizt auf Siedetemperatur, bevor er in der Kolonne eingespeist wurde. Das Rücklaufverhältnis betrug ca. 170. Die Destillation wurde kontinuierlich solange durchgeführt, bis die 35 kg Zulauf aufgebraucht wurden.

Im Seitenabzug wurde reines Cyclopentanon (im Mittel 313 g/h) mit einer Reinheit von 99,9% erhalten.

Am Kopf wurde neben einer geringen Menge Abgas ein flüssiges Produkt erhalten (im Mittel 6 g/h), das folgende Zusammensetzung aufwies: 4-Pentenal (52,6 Gew.-%), Cyclopentan (15,8 Gew.-%), Cyclopentenoxid (14,8 Gew.-%), 3-Methyl-2-butanon (10,4 Gew.-%), 3-Methylpentan (2,4 Gew.-%), Cyclopenten (1,7 Gew.-%), Methylcyclopentan (1,2 Gew.-%) neben einer Reihe von weiteren Nebenprodukten mit Konzentrationen jeweils unter 1000 ppm.

Insgesamt wurden aus der Destillation ca. 600 g des Kopfstroms erhalten.

### Beispiel 3: Aufkonzentrierung des Kopfstroms aus Beispiel 2

Das Kopfstromprodukt aus Beispiel 2 wurde in die Blase einer Batchdestillationskolonne eingefüllt. Die verwendete Kolonne hatte eine Höhe von 0,5 m und war mit Raschig-Ringen aus Metall gefüllt. Die Destillation wurde bei Normaldruck durchgeführt. Es wurde solange Produkt über Kopf abgezogen, bis die Kopftemperatur über 73°C stieg. Die Destillation wurde dann abgebrochen, der Sumpf abgekühlt und die Anlage mit N₂ inertisiert. Das Destillat (ca. 120 g) wurde verworfen.

Das im Sumpf verbleibende Produkt war nur leicht gelb gefärbt und enthielt laut GC-Analyse 4-Pentenal (67 %), Cyclopentenoxid (19 %), 3-Methyl-2-butanon (13 %) neben einer Reihe von weiteren Nebenprodukten mit Konzentrationen jeweils unter 2000 ppm.

Dieses Produkt wurde unter Stickstoff aufbewahrt und für die Oxidationsversuche ohne weitere Behandlung eingesetzt.

### Referenz Beispiel 4: Oxidation von 4-Pentenal (mit NaClO₂)

In einem 1000 ml-Rührkolben wurden 78,8 g (0,95 mol) 4-Pentenal in 370 ml Acetonitril vorgelegt. Innerhalb von 1,5 h wurden parallel 64,7 g (0,95 mol) wässrige Wasserstoffperoxid-Lösung (50 Gew.-% in Wasser) und eine Lösung von 107,4 g (0,95 mol) Natriumchlorit (technisch, ca. 80%ig) in 400 ml Wasser bei 25-40 °C zugetropft. Die einphasige Reaktionsmischung wurde für 1,5 h bei 25°C nachgerührt. Anschließend wurde der Ansatz dreimal mit 100 ml Dichlormethan extrahiert. Der pH-Wert der Phasen lag bei ca. 5-6. Die organische Phase wurde am Rotationsverdampfer eingeengt.

Es verblieben 58 g als Rückstand, der destillativ aufgereinigt wurde. Die Ausbeute an 4-Pentensäure mit einer Reinheit von 95% betrug 26 %.

Die so gewonnene Probe an 4-Pentensäure wurde mittels eines Riechstreifentest von einem Parfümeur geruchlich beurteilt. Der erste geruchliche Eindruck wurde als käsig, an Buttersäure erinnernd und säuerlich beschrieben. Nach 10 Minuten wurde der geruchliche Eindruck als käsig, nach Propionsäure riechend beschrieben. Nach 1 h wurde der Eindruck als käsig, etwas säuerlich beschrieben.

Zusätzlich wurde die Probe einem Gasraumtest unterzogen. Dazu wurde 1 ml der Probe in einem Twist-off-Glas mit 50 ml Wasser vermischt und das verschlossene Glas 10 Minuten ruhen gelassen. Anschließend wurde es geöffnet und die Gasphase abgerochen. Der geruchliche Eindruck wurde als fruchtig und angenehm beschrieben.

### Referenz Beispiel 5: Oxidation von 4-Pentenal (mit O₂)

In einer thermostatisierten Blasensäule mit Doppelmantel aus Glas (Innendurchmesser = 33 mm, Höhe H= 550 mm, unten mit einer P160 Glasfritte versehen und gefüllt mit Glas-Raschig-Ringen (5×5 mm)) wurden 151 g 4-Pentenal eingefüllt. Durch eine Glasfritte am Boden der Blasensäule wurden 5 Nl/h Sauerstoff (Nl = Norm-Liter) eingeperlt. Die Temperatur des Kühlmediums im Doppelmantel wurde über einen externen Thermostaten auf 20°C eingestellt. Der Gasraum am oberen Ende der Blasensäule wurde durch Spülung mit Stickstoff inertisiert. Nach 24 Stunden wurden die Reaktion abgebrochen und die Zusammensetzung des Reaktionsaustrags per GC ermittelt. Der Umsatz an 4-Pentenal betrug 49% und die Selektivität zu 4-Pentensäure ca. 82%.

Der Reaktionsaustrag wurde destillativ aufgearbeitet, um 4-Pentensäure mit einer Reinheit von 98,9 Gew.-% zu erhalten.

Die so gewonnene Probe an 4-Pentensäure wurde mittels eines Riechstreifentest von einem Parfümeur geruchlich beurteilt. Der erste geruchliche Eindruck wurde als säuerlich, an Propionsäure erinnernd und etwas käsig beschrieben. Nach 10 Minuten wurde der geruchliche Eindruck als schwach blumig, käsig und säuerlich beschrieben. Nach 1 h wurde der Eindruck als käsig, an Propionsäure erinnernd beschrieben.

Zusätzlich wurde die Probe einem Gasraumtest unterzogen. Dazu wurde 1 ml der Probe in einem Twist-off-Glas mit 50 ml Wasser vermischt und das verschlossene Glas 10 Minuten ruhen gelassen. Anschließend wurde es geöffnet und die Gasphase abgerochen. Der geruchliche Eindruck wurde als fruchtig und etwas staubig beschrieben.

### Beispiel 6: Oxidation von crude 4-Pentenal (mit O₂)

Es wurde wie im Beispiel 5 verfahren, wobei an Stelle von 4-Pentenal das Gemisch aus 4-Pentenal (67 %), Cyclopentenoxid (19 %), 3-Methyl-2-butanon (13 %) aus Beispiel 3 eingesetzt wurde. Davon wurden 150 g in die Blasensäule eingefüllt. Ansonsten so wurde verfahren wie in Beispiel 5.

Nach 70 Stunden Reaktionszeit wurde der Austrag mittels GC analysiert. Der Umsatz an 4-Pentenal betrug 32% und die Selektivität zu 4-Pentensäure 82%. Die Umsätze an Cyclopentenoxid und 3-Methyl-2-butanon waren vernachlässigbar klein (<5%), und es konnten keine daraus abgeleiteten Produkten (wie z.B. 3-Hydroxy-3-methyl-2-butanon) im GC nachgewiesen werden. Der Aktivsauerstoffgehalt (iodometrisch bestimmt) betrug lediglich 5 g/kg Lösung.

Der Reaktionsaustrag wurde destilliert, wobei 4-Pentensäure mit einem Gehalt von 98 Gew.-% gewonnen werden konnte. Vom geruchlichen Eindruck war die 4-Pentensäure vergleichbar zu der, die in Beispiel 5 aus der Oxidation von reinem 4-Pentenal gewonnen wurde.

Dieses Beispiel zeigt, dass auch beim Einsatz des Rohgemisches die Oxidation von 4-Pentenal selektiv möglich ist und die Qualität der daraus gewonnene 4-Pentensäure nicht beeinträchtigt wird.

## Patentansprüche

1. Verfahren zur Herstellung von 4-Pentensäure, mindestens umfassend den Schritt
(a) Oxidation eines Gemischs (G), enthaltend 4-Pentenal, 3-Methyl-2-butanon und Cyclopentenoxid,
wobei die Oxidation gemäß Schritt (a) in Abwesenheit eines Katalysators durchgeführt wird.

2. Verfahren nach Anspruch 1, wobei das Gemisch (G) 10 bis 90 Gew.-% 4-Pentenal enthält.

3. Verfahren nach Anspruch 1 oder 2, wobei für die Oxidation gemäß Schritt (a) ein sauerstoffhaltiges Gasgemisch als Oxidationsmittel eingesetzt wird.

4. Verfahren nach einem der Ansprüche 1 bis 3, wobei die Oxidation gemäß Schritt (a) in Gegenwart eines Lösungsmittels durchgeführt wird.

5. Verfahren nach einem der Ansprüche 1 bis 4, wobei die Oxidation gemäß Schritt (a) in Gegenwart eines Lösungsmittels ausgewählt aus der Gruppe bestehend aus 4-Pentensäure, 2-Ethylhexansäure, Isononansäure, Propylheptansäure und Neodecansäure durchgeführt wird.

6. Verfahren nach einem der Ansprüche 1 bis 5, wobei das Gemisch (G) als Nebenprodukt der Oxidation von Cyclopenten zu Cyclopentanon erhalten wird.

7. Verfahren nach Anspruch 6, wobei die Oxidation von Cyclopenten zu Cyclopentanon in der Gegenwart von Distickstoffmonoxid erfolgt.

## Claims

1. A process for preparing 4-pentenoic acid, at least comprising the step of
(a) oxidizing a mixture (G) comprising 4-pentenal, 3-methyl-2-butanone and cyclopentene oxide,
wherein the oxidation in step (a) is performed in the absence of a catalyst.

2. The process according to claim 1, wherein the mixture (G) comprises 10 to 90% by weight of 4-pentenal.

3. The process according to claim 1 or 2, wherein an oxygenous gas mixture is used as the oxidizing agent for the oxidation in step (a).

4. The process according to any of claims 1 to 3, wherein the oxidation in step (a) is performed in the presence of a solvent.

5. The process according to any of claims 1 to 4, wherein the oxidation in step (a) is performed in the presence of a solvent selected from the group consisting of 4-pentenoic acid, 2-ethylhexanoic acid, isononanoic acid, propylheptanoic acid and neodecanoic acid.

6. The process according to any of claims 1 to 5, wherein the mixture (G) is obtained as a by-product of the oxidation of cyclopentene to cyclopentanone.

7. The process according to claim 6, wherein cyclopentene is oxidized to cyclopentanone in the presence of dinitrogen monoxide.

## Revendications

1. Procédé pour la production d'acide 4-penténoïque, au moins comprenant l'étape
(a) oxydation d'un mélange (G) contenant du 4-penténal, de la 3-méthyl-2-butanone et de l'oxyde de cyclopentène,
dans lequel on effectue l'oxydation selon l'étape (a) en absence d'un catalyseur

2. Procédé selon la revendication 1, dans lequel le mélange (G) contient de 10 à 90 % en poids de 4-penténal.

3. Procédé selon la revendication 1 ou 2, dans lequel pour l'oxydation selon l'étape (a) on utilise comme oxydant un mélange de gaz contenant de l'oxygène.

4. Procédé selon l'une quelconque des revendications 1 à 3, dans lequel on effectue l'oxydation selon l'étape (a) en présence d'un solvant.

5. Procédé selon l'une quelconque des revendications 1 à 4, dans lequel on effectue l'oxydation selon l'étape (a) en présence d'un solvant choisi dans le groupe constitué par l'acide 4-penténoïque, l'acide 2-éthylhexanoïque, l'acide isononaoïque, l'acide propylheptanoïque et l'acide néodécanoïque.

6. Procédé selon l'une quelconque des revendications 1 à 5, dans lequel le mélange (G) est obtenu en tant que sous-produit de l'oxydation du cyclopentène en cyclopentanone.

7. Procédé selon la revendication 6, dans lequel l'oxydation du cyclopentène en cyclopentanone s'effectue en présence de protoxyde d'azote.
